# NEW EUROPEAN PATENT SPECIFICATION

(11) **EP 3 475 702 B3**
(45) Date of publication of this specification: **20.03.2024**
(45) Mention of the grant of the patent: 14.06.2023
(21) Application number: 17814901.9
(22) Date of filing: 23.06.2017
(51) Int. Cl.: G01N 33/543, G01N 33/569, C12Q 1/04, B01L 99/00

(54) **MEASURING TRAIL BY LATERAL FLOW IMMUNOASSAY**
TRAIL-MESSUNG DURCH LATERAL-FLOW-IMMUNOASSAAY
MESURE DE TRAIL PAR IMMUNOESSAI SUR MEMBRANE

(30) Priority: 23.06.2016 US 201662353697 P
(43) Date of publication of application: 01.05.2019
(73) Proprietor: Memed Diagnostics Ltd., 3508504 Tirat HaCarmel (IL)
(72) Inventor: OVED, Kfir, 3087500 Hof HaCarmel (IL); EDEN, Eran, 3498233 Haifa (IL); BOICO, Olga, 3497203 Haifa (IL); KRONENFELD, Gali, 3220503 Tirat Carmel (IL); NAVON, Roy, 6423313 Tel-Aviv (IL); COHEN-DOTAN, Assaf, 4265930 Natania (IL)
(74) Representative: Gregson, Anna Louise
(86) International application number: PCT/IL2017/050697
(87) International publication number: WO 2017/221255

(56) References cited:
- WO-A1-2013/117746
- WO-A1-2013/117746
- WO-A1-2016/024278
- WO-A2-2010/033963
- WO-A2-2010/033963
- LEUNG W ET AL: "InfectCheck CRP barcode-style lateral flow assay for semi-quantitative detection of C-reactive protein in distinguishing between bacterial and viral infections", JOURNAL OF IMMUNOLOGICAL METHODS, ELSEVIER SCIENCE PUBLISHERS B.V.,AMSTERDAM, NL, vol. 336, no. 1, 20 July 2008 (2008-07-20) , pages 30-36, XP022682288, ISSN: 0022-1759, DOI: 10.1016/J.JIM.2008.03.009 [retrieved on 2008-04-10]
- LEUNG, W. ET AL.: 'InfectCheck CRP barcode-style lateral flow assay for semi-quantitative detection of C-reactive protein in distinguishing between bacterial and viral infections' JOURNAL OF IMMUNOLOGICAL METHODS vol. 336, 10 April 2008, pages 30 - 36, XP022682288
- SUTHERLAND, J.S. ET AL.: 'Use of lateral flow assays to determine IP-10 and CCL4 levels in pleural effusions and whole blood for TB diagnosis' TUBERCULOSIS vol. 96, 14 November 2015, pages 31 - 6, XP029389997

## Description

### FIELD AND BACKGROUND OF THE INVENTION

The present invention, in some embodiments thereof, relates to methods of measuring TRAIL and CRP polypeptides using lateral flow immunoassays. The assay can be used for a myriad of different applications including distinguishing between bacterial and viral infections and overall prognosis of patients.

Antibiotics (Abx) are the world's most prescribed class of drugs with a 25-30 billion $US global market. Abx are also the world's most misused drug with a significant fraction of all drugs (40-70%) being wrongly prescribed.

One type of Abx misuse is when the drug is administered in case of a non-bacterial disease, such as a viral infection, for which Abx is ineffective. For example, according to the USA center for disease control and prevention CDC, over 60 Million wrong Abx prescriptions are given annually to treat flu in the US. The health-care and economic consequences of the Abx over-prescription include: (i) the cost of antibiotics that are unnecessarily prescribed globally, estimated at >$10 billion annually; (ii)side effects resulting from unnecessary Abx treatment are reducing quality of healthcare, causing complications and prolonged hospitalization (e.g. allergic reactions, Abx associated diarrhea, intestinal yeast etc.) and (iii) the emergence of resistant strains of bacteria as a result of the overuse (the CDC has declared the rise in antibiotic resistance of bacteria as "one of the world's most pressing health problems in the 21^{st} century".

Antibiotics under-prescription is not uncommon either. For example up to 15% of adult bacterial pneumonia hospitalized patients in the US receive delayed or no Abx treatment, even though in these instances early treatment can save lives and reduce complications.

Technologies for infectious disease diagnostics have the potential to reduce the associated health and financial burden associated with Abx misuse. Ideally, such a technology should: (i) accurately differentiate between a bacterial and viral infections; (ii) be rapid (within minutes); (iii) be able to differentiate between pathogenic and non-pathogenic bacteria that are part of the body's natural flora; (iv) differentiate between mixed co-infections and pure viral infections and (v) be applicable in cases where the pathogen is inaccessible (e.g. sinusitis, pneumonia, otitis-media, bronchitis, etc).

Current solutions (such as culture, PCR and immunoassays) do not fulfill all these requirements: (i) Some of the assays yield poor diagnostic accuracy (e.g. low sensitivity or specificity), and are restricted to a limited set of bacterial or viral strains; (ii) they often require hours to days; (iii) they do not distinguish between pathogenic and non-pathogenic bacteria, thus leading to false positives; (iv) they often fail to distinguish between a mixed and a pure viral infections and (v) they require direct sampling of the infection site in which traces of the disease causing agent are searched for, thus prohibiting the diagnosis in cases where the pathogen resides in an inaccessible tissue, which is often the case.

Consequentially, there is still a diagnostic gap, which in turn often leads physicians to either over-prescribe Abx (the "Just-in-case-approach"), or under-prescribe Abx (the "Wait-and-see-approach"), both of which have far reaching health and financial consequences.

Accordingly, a need exists for a rapid method that accurately differentiates between bacterial, viral, mixed and non-infectious disease patients that addresses these challenges.

### Background art includes:

WO 2013/117746 A1 discloses methods of determining the presence or absence of a bacterial and/or viral infection in a subject, comprising measuring the concentration of TRAIL in a sample derived from the subject.

WO 2016/024278 A1 discloses methods of analyzing biological data containing expression values of a plurality of polypeptides in the blood of a subject.

WO 2010/033963A2 discloses methods of determining if an infection if bacterial or viral comprising the detection of CRP.

Leung et al. (2008), Journal of Immunological Methods, 336(1): 30-36, describes an InfectCheck barcode-style lateral flow assay for semi-quantitative detection of CRP in distinguishing between bacterial and viral infections.

### SUMMARY OF THE INVENTION

The technical information set out below may in some respects go beyond the scope of the claimed invention. The additional technical information is provided to place the claimed invention in a broader technical context and to illustrate possible related technical developments.

According to one aspect of the present invention, there is provided a method of measuring the amount of TNF-related apoptosis-inducing ligand (TRAIL) polypeptide and CRP polypeptide in a fluid sample of a subject in need thereof, the method comprising:
(a) flowing said sample through a lateral flow immunoassay (LFI) device which comprises a first mobile, labeled monoclonal antibody against said TRAIL; a second mobile labeled monoclonal antibody against said CRP; a first immobilized, non-labeled monoclonal antibody against said TRAIL; and a second immobilized, non-labeled monoclonal antibody against said CRP; and
(b) determining the amount of said TRAIL polypeptide and said CRP polypeptide at a test band of said device.

According to another aspect of the present invention, there is provided a method of distinguishing between a bacterial and viral infection in a subject comprising:
(a) measuring the amount of TRAIL polypeptide and CRP polypeptide in a fluid sample of the subject according to the method of the invention; and
(b) diagnosing the subject with a bacterial or viral infection according to the amount of said TRAIL polypeptide and said CRP polypeptide.

According to another aspect of the present invention, there is a lateral flow test strip comprising a conjugate pad which comprises:
(a) a first mobile, labeled monoclonal antibody against TRAIL polypeptide and a detection membrane comprising an immobilized, non-labeled monoclonal antibody against said TRAIL polypeptide; and
(b) a second mobile, labeled monoclonal antibody against CRP polypeptide and a detection membrane comprising an immobilized, non-labeled monoclonal antibody against said CRP polypeptide.

According to an aspect of some embodiments of the present invention, there is provided a kit comprising the lateral flow test strip as claimed, a sample collector and a diluent.

Further embodiments of the claimed invention are listed in the appended claims

According to some embodiments of the invention, the test band comprises an immobilized capture reagent capable of binding to the labeled unbound antibody but not to the immunocomplex.

According to some embodiments of the invention, the test band comprises an immobilized capture reagent capable of binding to the TRAIL polypeptide of the immunocomplex and not to the labeled antibody.

According to some embodiments of the invention, the labeled antibody comprises a label selected from the group consisting of a chromogen, a catalyst, a fluorescent agent, a chemiluminescent agent, a dye particle, and a latex particle tagged with a detector reagent.

According to some embodiments of the invention, the lateral flow immunoassay device is capable of detecting at least one additional polypeptide, the polypeptide being selected from the group consisting of CRP, IP10, NGAL, MMP8, IL1RA, OTOF, PI3, CYBRD1, EIF2AK2, CMPK2, Mac-2BP, B2M, BCA-1, CHI3L1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, SAA, TREM-1, PCT, IL-8, IL6, ARG1, ARPC2, ATP6V0B, BCA-1, BRI3BP, CCL19-MIP3b, CES1, CORO1A, HERC5, IFI6, IFIT3, KIAA0082, LIPT1, LRDD, MCP-2, PARP9, PTEN, QARS, RAB13, RPL34, SART3, TRIM22, UBE2N, XAF1 and ZBP1.

According to some embodiments of the invention, the at least one additional polypeptide is IP10.

According to some embodiments of the invention, the lateral flow test strip comprises a labeled antibody against the at least one additional polypeptide.

According to some embodiments of the invention, the lateral flow immunoassay device comprises an additional lateral flow test strip which comprises a labeled antibody against the at least one additional polypeptide.

According to some embodiments of the invention, the lateral flow test strip comprises a labeled antibody against the IP10.

According to some embodiments of the invention, the lateral flow immunoassay device comprises an additional lateral flow test strip which comprises a labeled antibody against the CRP.

According to some embodiments of the invention, the lateral flow immunoassay device comprises an additional lateral flow test strip which comprises a labeled antibody against the IP10.

According to some embodiments of the invention, the lateral flow immunoassay device comprises a first lateral flow test strip which comprises a labeled antibody against the TRAIL, a second lateral flow test strip which comprises a labeled antibody against the CRP and a third lateral flow test strip which comprises a labeled antibody against the IP10.

Accordingto some embodiments of the invention, the fluid sample comprises a blood sample or a fraction thereof.

According to some embodiments of the invention, the labeled antibody comprises a label selected from the group consisting of a chromogen, a catalyst, a gold nanoparticle, a fluorescent agent, a chemiluminescent agent, a dye particle, and a latex particle tagged with a detector reagent.

According to some embodiments of the invention, the lateral flow immunoassay device is capable of detecting at least one additional polypeptide, the polypeptide being selected from the group consisting of IP10, NGAL, MMP8, IL1RA, OTOF, PI3, CYBRD1, EIF2AK2, CMPK2, Mac-2BP, B2M, BCA-1, CHI3L 1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, SAA, TREM-1, PCT, IL-8, IL6, ARG1, ARPC2, ATP6V0B, BCA-1, BRI3BP, CCL19-MIP3b, CES1, CORO1A, HERC5, IFI6, IFIT3, KIAA0082, LIPT1, LRDD, MCP-2, PARP9, PTEN, QARS, RAB13, RPL34, SART3, TRIM22, UBE2N, XAF1 and ZBP1.

According to some embodiments of the invention, the at least one additional polypeptide is IP10.

According to some embodiments of the invention, the first labeled antibody and the second labeled antibody are comprised on a single lateral flow test strip.

According to some embodiments of the invention, the first labeled antibody is comprised on a first lateral flow test strip and the second labeled antibody is comprised on a second lateral flow test strip.

According to some embodiments of the invention, the first or the second lateral flow test strip comprises a labeled antibody against IP10.

According to some embodiments of the invention, the lateral flow immunoassay device comprises an additional lateral flow test strip which comprises a labeled antibody against the IP10.

According to some embodiments of the invention, the lateral flow immunoassay device comprises a first lateral flow test strip which comprises a labeled antibody against the TRAIL, a second lateral flow test strip which comprises a labeled antibody against the CRP and a third lateral flow test strip which comprises a labeled antibody against the IP10.

Accordingto some embodiments of the invention, the fluid sample comprises a blood sample or a fraction thereof.

According to some embodiments of the invention, the fluid sample has been taken from the subject no more than two days following symptoms of an infection.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

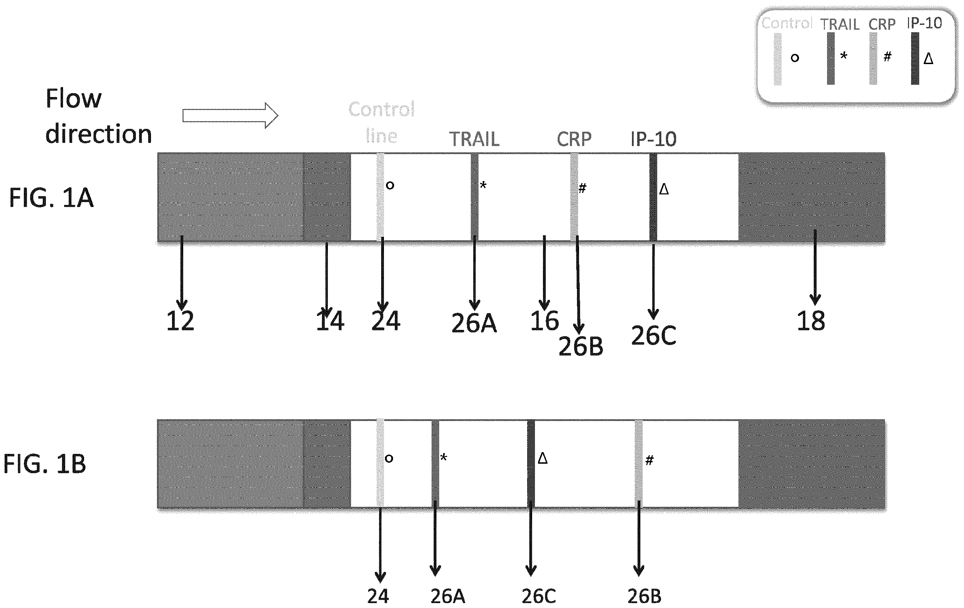

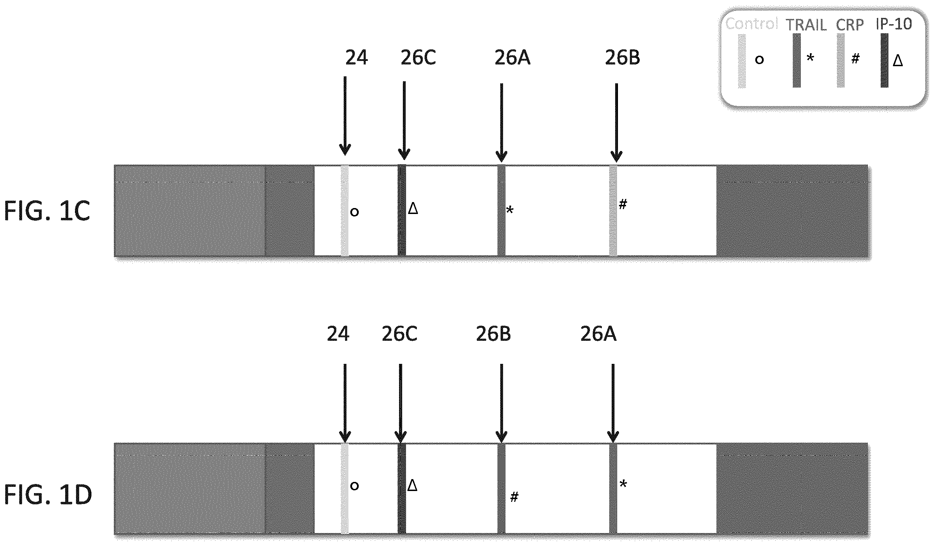

FIGs. 1A-F are schematic illustrations of test strips configured to be used in a lateral flow immunoassay according to embodiments of the present invention. In these embodiments, a single strip is used which is able to detect three polypeptide determinants - TRAIL, CRP and IP-10. In each of the illustrations a different order of the antibodies which recognize the polypeptide determinants is shown.

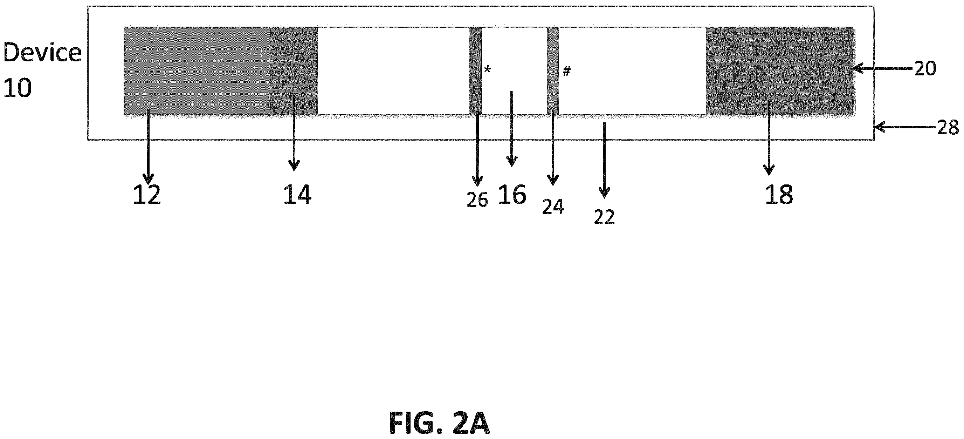

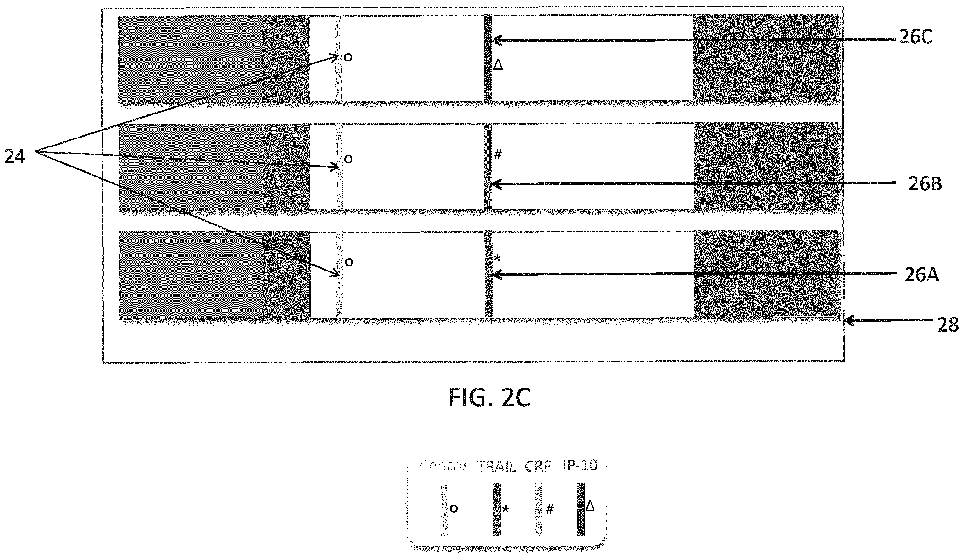

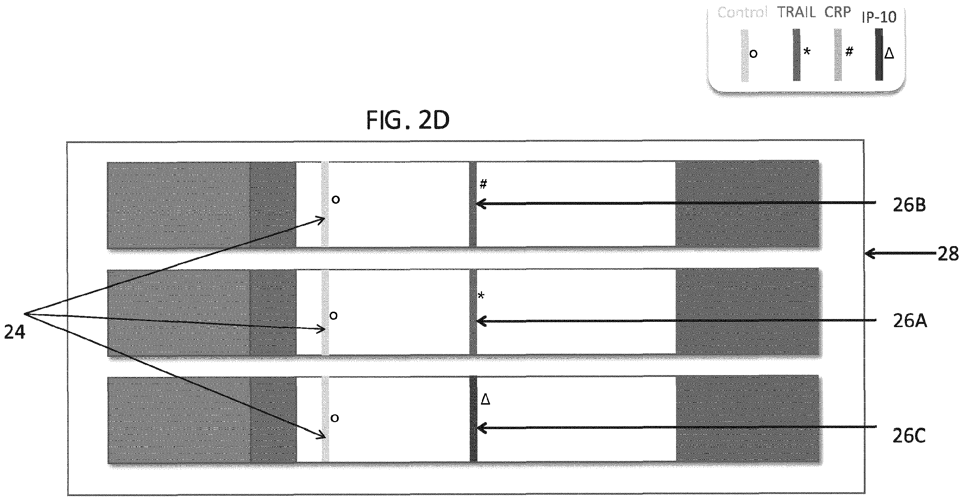

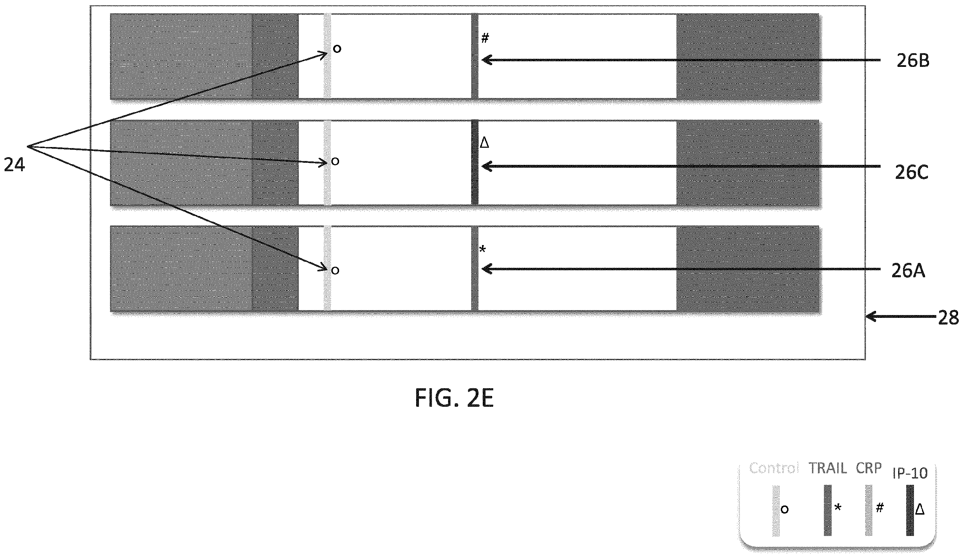

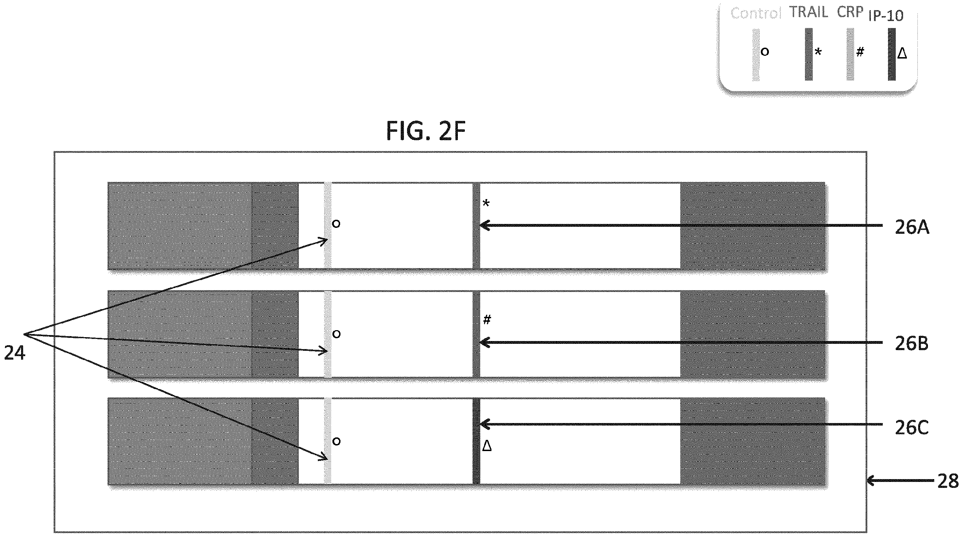

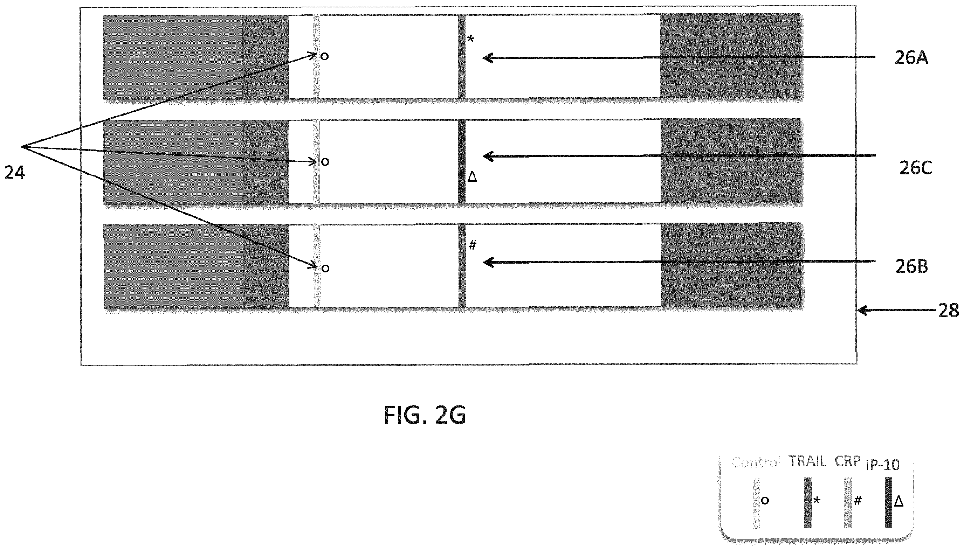

FIGs. 2A-G are schematic illustrations of test strips configured to be used in a lateral flow immunoassay according to embodiments of the present invention. In these embodiments, three strips are used per test, each strip for detection of a single polypeptide determinant. In each of the illustrations a different order of strips is shown. Figure 2A annotates the different parts of the strips.

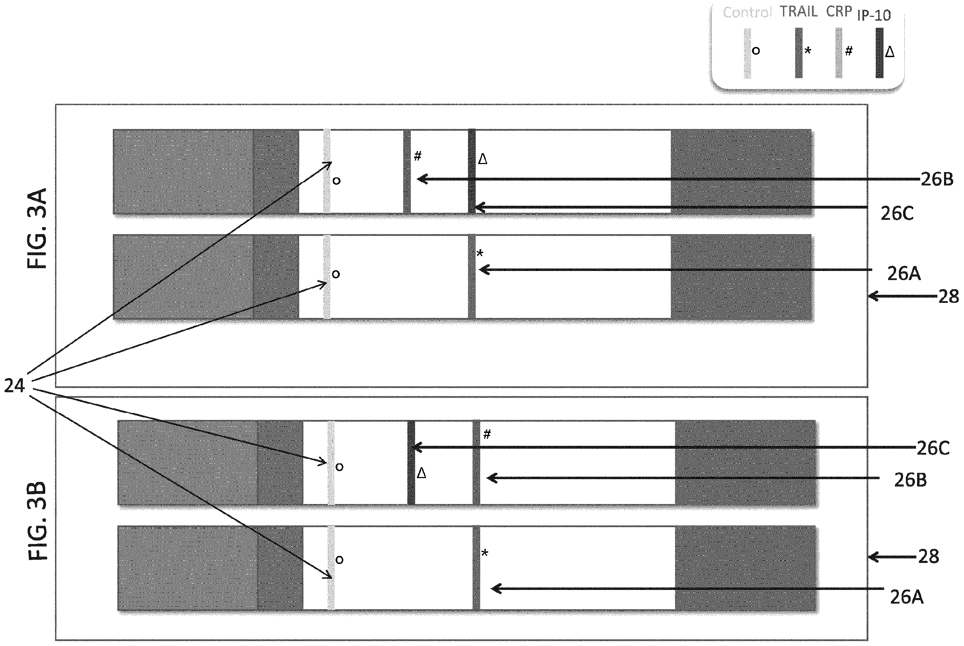

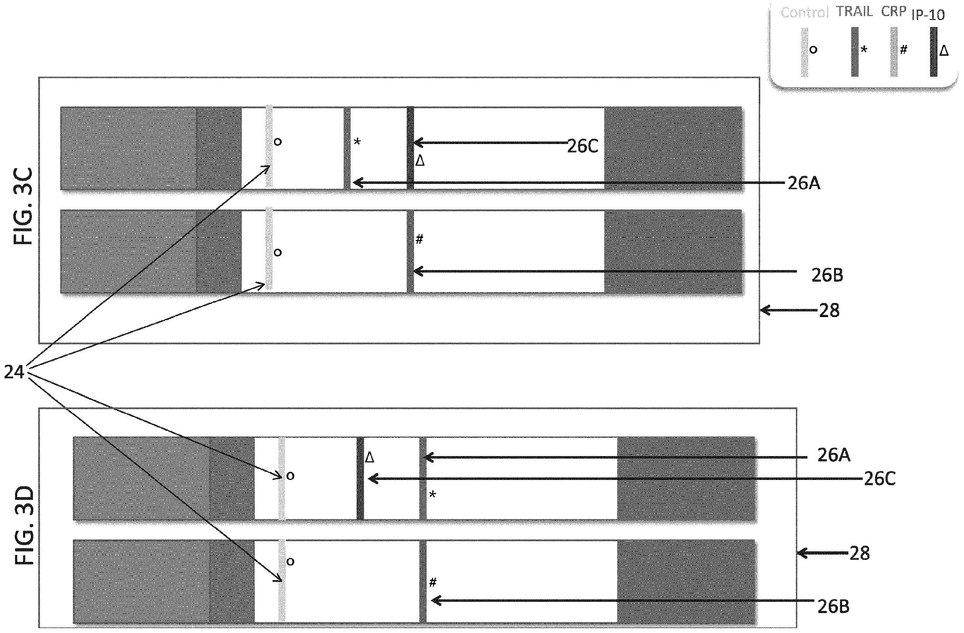

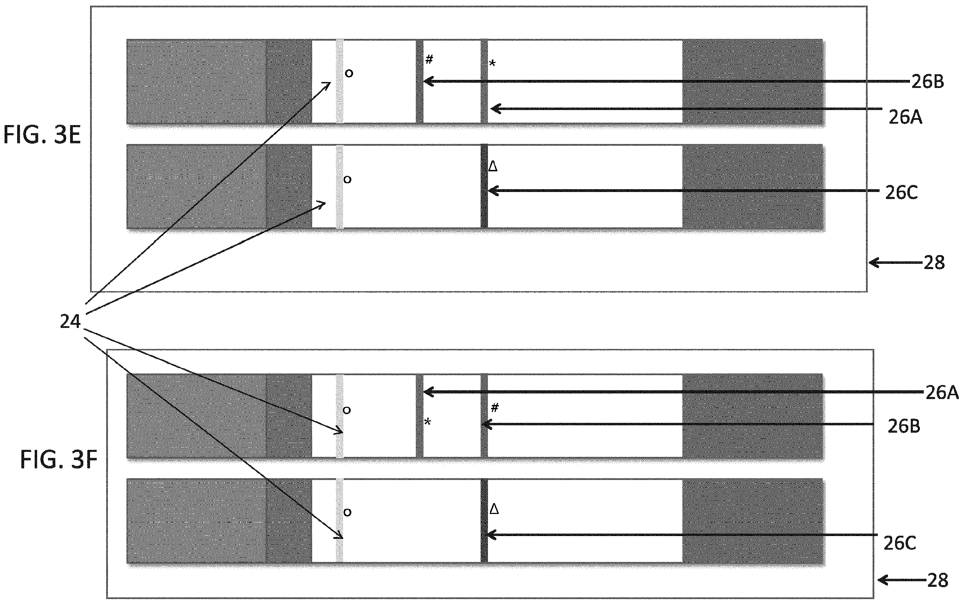

FIGs. 3A-F are schematic illustrations of test strips configured to be used in a lateral flow immunoassay according to embodiments of the present invention. In these embodiments, two strips are used per test, one strip for detection of a single polypeptide determinant and the other strip for detection of two polypeptide determinants. In each of the illustrations a different order of strips and/or antibodies is shown.

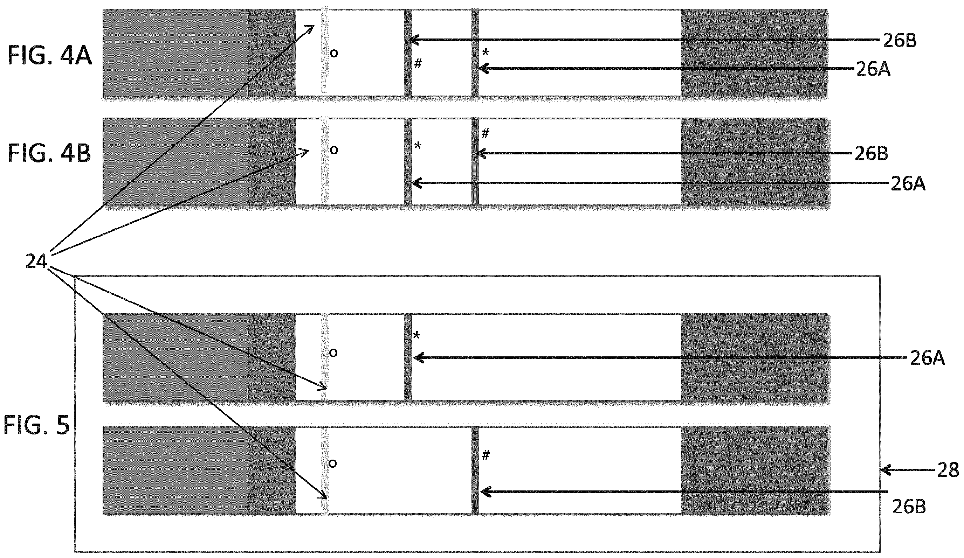

FIGs. 4A-B are schematic illustrations of test strips configured to be used in a lateral flow immunoassay according to embodiments of the present invention. In these embodiments, a single strip is used which is able to detect two polypeptide determinants - TRAIL, and CRP. In each of the illustrations a different order of the antibodies which recognize the polypeptide determinants is shown.
FIG. 5 is a schematic illustration of test strips configured to be used in a lateral flow immunoassay according to embodiments of the present invention. In these embodiments, two strips are used per test, each strip for detection of a single polypeptide determinant.

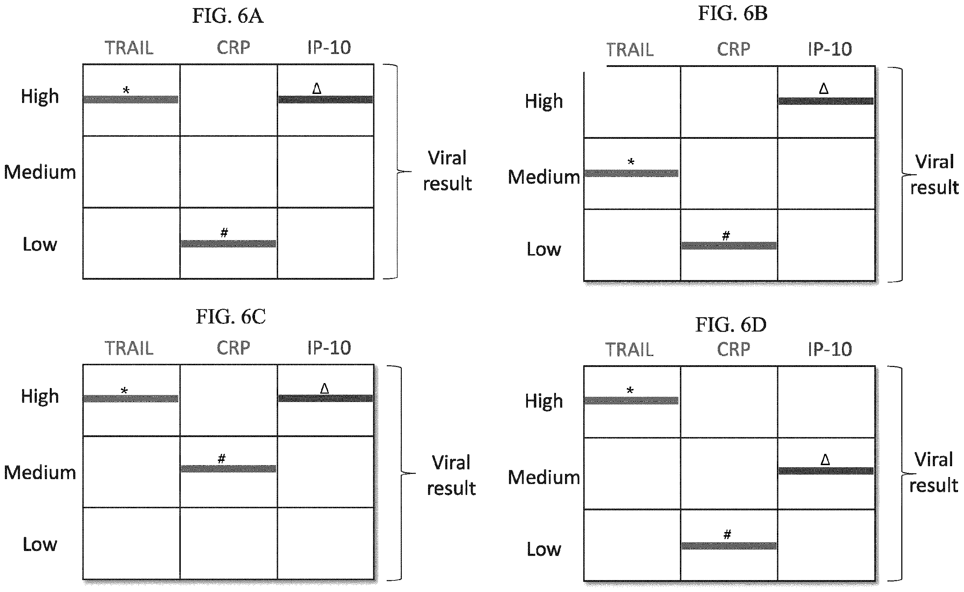

FIGs. 6A-D are schematic illustrations of exemplary readouts which may be obtained using a lateral flow immunoassay according to embodiments of the present invention which would indicate a viral infection using three predetermined levels, low, medium and high and three polypeptide determinants.

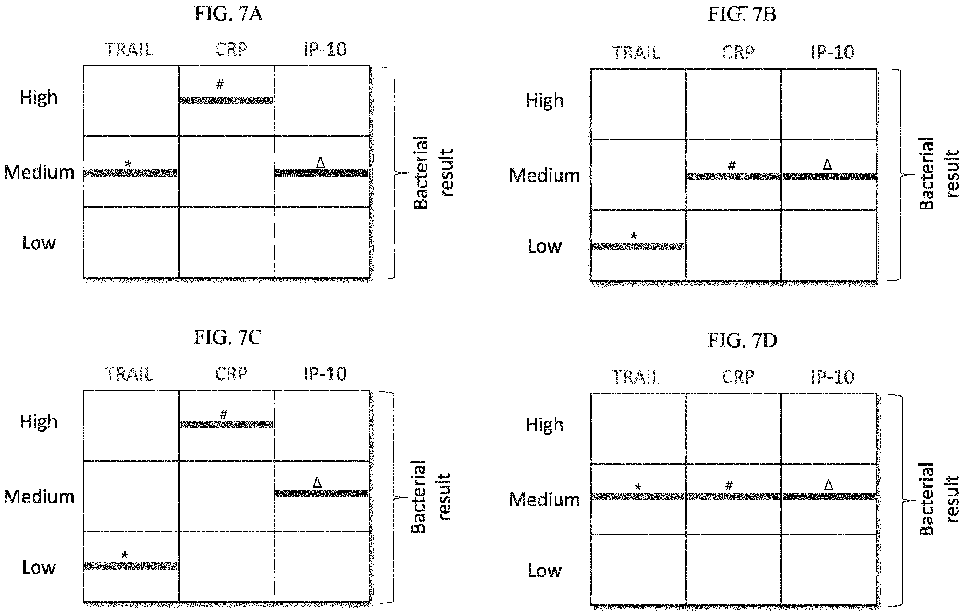

FIGs. 7A-D are schematic illustrations of exemplary readouts which may be obtained using a lateral flow immunoassay according to embodiments of the present invention which would indicate a bacterial infection, using three predetermined levels, low, medium and high and three polypeptide determinants.

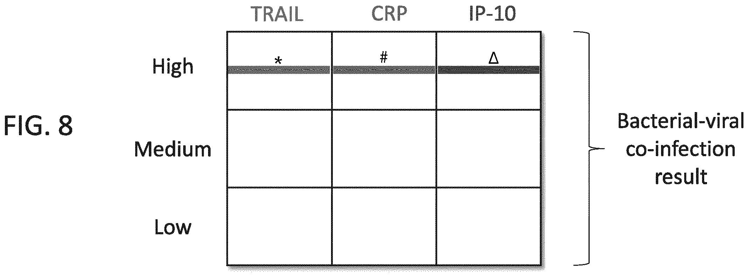

FIG. 8 are schematic illustrations of exemplary readouts which may be obtained using a lateral flow immunoassay according to embodiments of the present invention which would indicate a mixed bacterial/viral infection, using three predetermined levels, low, medium and high and three polypeptide determinants.

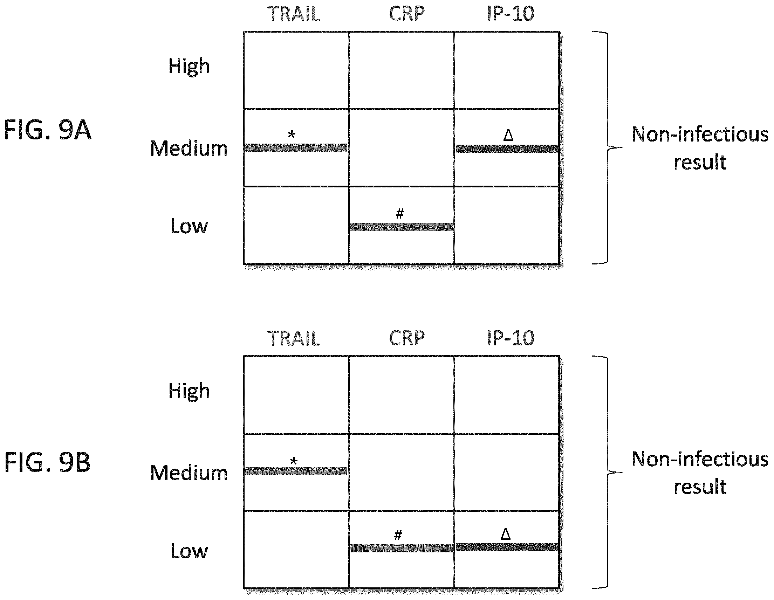

FIGs. 9A-B are schematic illustrations of exemplary readouts which may be obtained using a lateral flow immunoassay according to embodiments of the present invention which would indicate a non-infectious state, using three predetermined levels, low, medium and high and three polypeptide determinants.

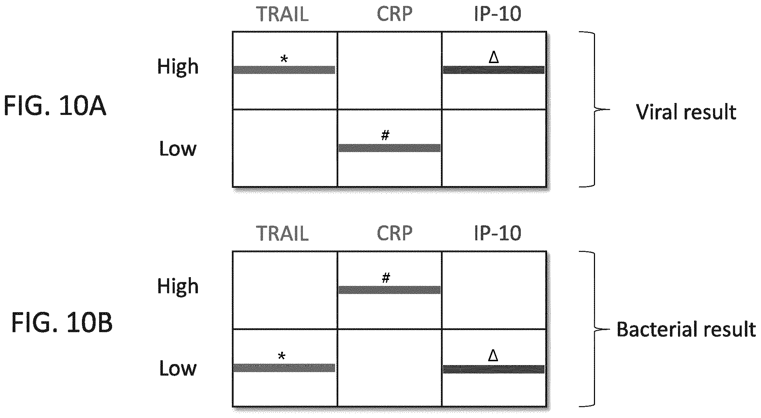

FIGs. 10A-D are schematic illustrations of exemplary readouts which may be obtained using a lateral flow immunoassay according to embodiments of the present invention which would indicate either a bacterial, viral infection or mixed infection using two predetermined levels, low, and high and three polypeptide determinants.

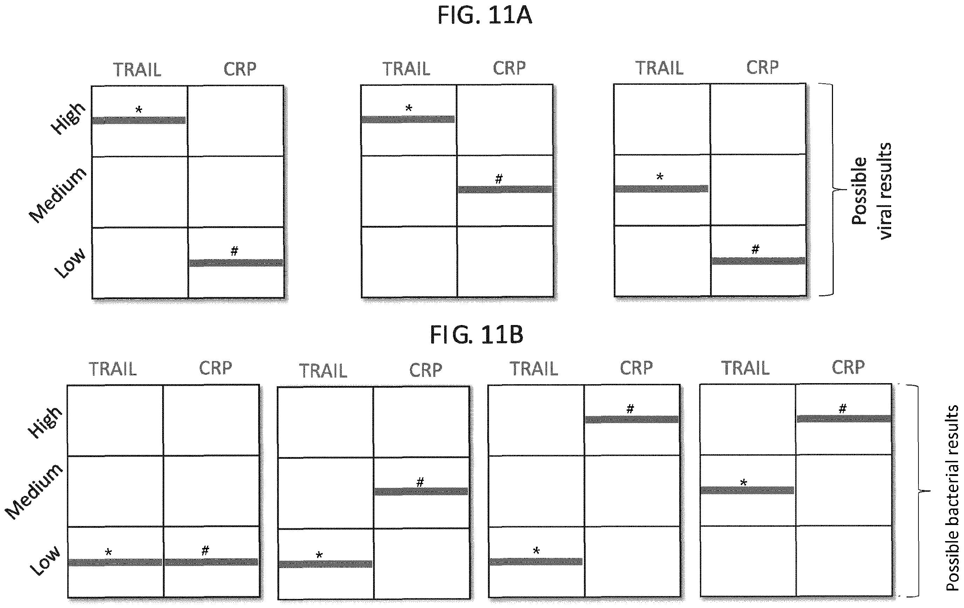

FIGs. 11A-C are schematic illustrations of exemplary readouts which may be obtained using a lateral flow immunoassay according to embodiments of the present invention which would indicate either a bacterial, viral infection or mixed infection using three predetermined levels, low, medium and high and two polypeptide determinants.

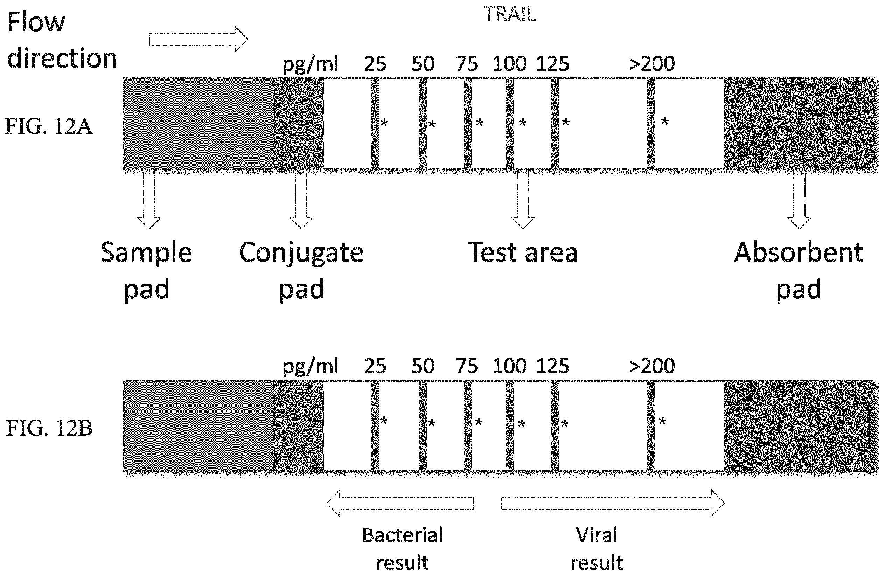

FIGs. 12A-B are schematic illustrations of exemplary readouts which may be obtained using a lateral flow immunoassay according to embodiments of the present invention which would indicate either a bacterial or viral infection based on predetermined levels of TRAIL.

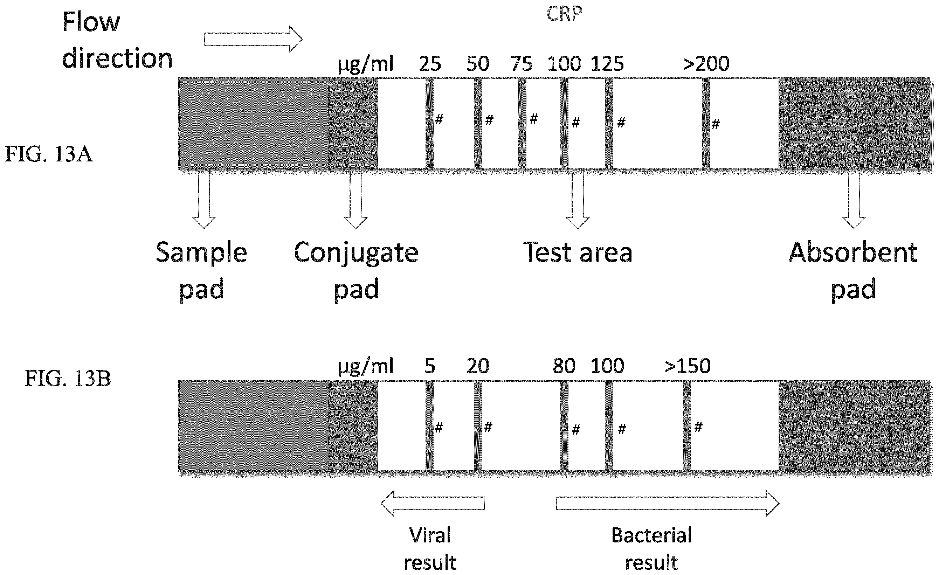

FIGs. 13A-B are schematic illustrations of exemplary readouts which may be obtained using a lateral flow immunoassay according to embodiments of the present invention which would indicate either a bacterial or viral infection based on predetermined levels of CRP.

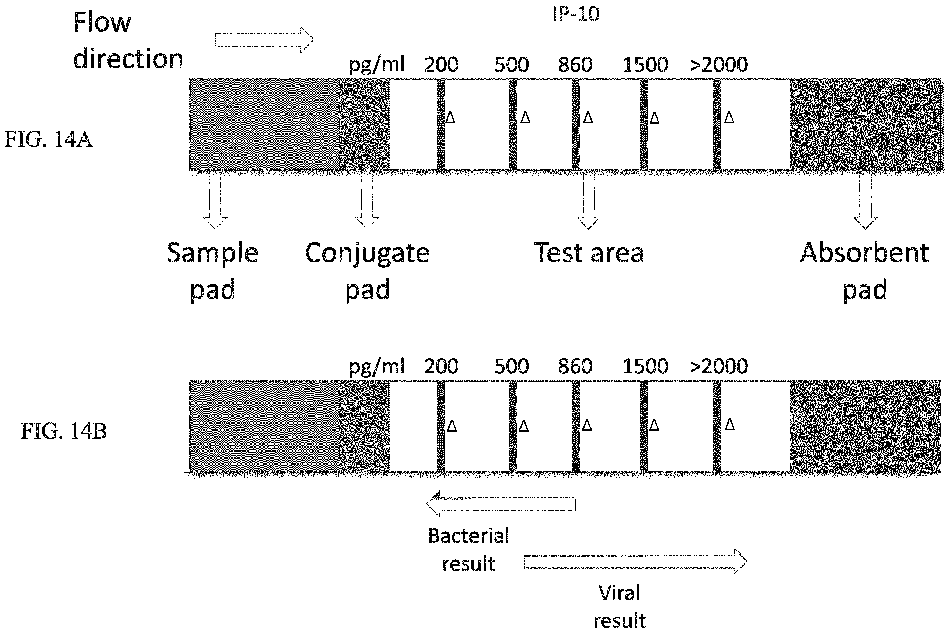

FIGs. 14A-B are schematic illustrations of exemplary readouts which may be obtained using a lateral flow immunoassay according to embodiments of the present invention which would indicate either a bacterial or viral infection based on predetermined levels of IP-10.

### DESCRIPTION OF SPECIFIC EMBODIMENTS OF THE INVENTION

The technical information set out below may in some respects go beyond the scope of the claimed invention. The additional technical information is provided to place the claimed invention in a broader technical context and to illustrate possible related technical developments.

The present invention, in some embodiments thereof, relates to methods of measuring TRAIL and CRP polypeptides using lateral flow immunoassays. The assay can be used for a myriad of different applications including distinguishing between bacterial and viral infections and overall prognosis of patients.

The present inventors previously discovered that TNF-related apoptosis-inducing ligand (TRAIL) levels are decreased in bacterial patients and increased in viral patients compared to non-infectious subjects. Based on their findings, they suggested TRAIL as a diagnostic marker for distinguishing between bacterial and viral patients (e.g. WO 2013/117746).

The present inventors now propose that the lateral flow immunoassay is an optimal method for analyzing the amount of TRAIL from a fluid sample of a subject, whether TRAIL is the single determinant being measured or whether a combination of determinants are measured, one of them being TRAIL.

Described is a method of measuring the amount of TNF-related apoptosis-inducing ligand (TRAIL) polypeptide in a fluid sample of a subject in need thereof using a lateral flow immunoassay (LFI) device, the method comprising:
(a) contacting the fluid sample with a lateral flow test strip which comprises a conjugate pad, the conjugate pad comprising a labeled antibody against the TRAIL polypeptide, wherein the contacting is effected under conditions that allow for the formation of an immunocomplex between the labeled antibody and the TRAIL polypeptide of the sample;
(b) flowing the unbound labeled antibody or immunocomplex through a test band; and
(c) determining the amount of the TRAIL polypeptide by analyzing the amount of either the unbound labeled antibody or the immunocomplex at the test band. The invention as claimed does not relate to measuring the amount of TRAIL in isolation, but rather measuring the amount of both TRAIL and CRP.

Lateral flow immunoassays are tests for the detection of the presence or absence of a target analyte in a sample for home testing, point of care testing, or laboratory applications. Lateral flow test strips utilize a solid support through which a mobile phase (e.g., a liquid sample) can flowthrough by capillary action to a reaction matrix 5 where a detectable signal, such as color changes or color differences on the test strip, may be generated to indicate the presence or absence of the target analyte.

As used herein, the term "capillary action" or "capillarity" refers to the process by which a molecule is drawn across the lateral test strip due to such properties as surface tension and attraction between molecules.

For any of the aspects disclosed herein, the term "measuring" or "measurement," or alternatively "detecting" or "detection," means assessing the presence, absence, quantity or amount (which can be an effective amount) of the determinant within a clinical or subject-derived sample, including the derivation of qualitative or quantitative concentration levels of such determinants.

**TRAIL:** The protein encoded by this gene is a cytokine that belongs to the tumor necrosis factor (TNF) ligand family. The present invention contemplates measuring either the soluble and/or the membrane form of this protein. In a particular embodiment, the soluble form of TRAIL is measured. Additional names of the gene include without limitations APO2L, TNF-related apoptosis-inducing ligand, TNFSF10 and CD253. This protein binds to several members of the TNF receptor superfamily such as TNFRSF10A/TRAILR1, TNFRSF10B/TRAILR2, TNFRSF10C/TRAILR3, TNFRSF10D/TRAILR4, and possibly also to TNFRSF11B/OPG.

Additional information concerning TRAIL is provided in Table 1, herein below.

**Table 1**

| **Protein symbol** | **Full Gene Name** | **RefSeq DNA sequence** | **RefSeq proteins** |
|---|---|---|---|
| TRAIL | Tumor necrosis factor superfamily member 10 | NC_000003.12 | NP_001177871.1 |
| | | NC_018914.2 | NP_001177872.1 |
| | | NT_005612.17 | NP_003801.1 |

A "subject" in the context of the present invention may be a mammal (e.g. a human, dog, cat, horse, cow, sheep, pig, goat). According to another embodiment, the subject is a bird (e.g. chicken, turkey, duck or goose). According to a particular embodiment, the subject is a human. The subject may be male or female. The subject may be an adult (e.g. older than 18, 21, or 22 years or a child (e.g. younger than 18, 21 or 22 years). In another embodiment, the subject is an adolescent (between 12 and 21 years), an infant (29 days to less than 2 years of age) or a neonate (birth through the first 28 days of life).

In one embodiment, the subject of this aspect of the present invention presents with symptoms of a disease - e.g. infectious disease.

In another embodiment, the subject of this aspect of the present invention does not show symptoms of a disease (i.e. asymptomatic).

Exemplary symptoms of infectious diseases include but are not limited to fever, nausea, headache, sore throat, runny nose, rash and/or muscle soreness. According to a particular embodiment, the symptoms of an infectious disease includes fever.

According to a particular embodiment, the subject does not show signs of having had a heart attack (e.g. has a normal level of creatine kinase, troponin or serum myoglobin, and/or has a normal ECG or EKG).

According to yet another embodiment, the subject does not have cancer.

A "sample" in the context of the present invention is a biological sample isolated from a subject and can include, by way of example and not limitation, whole blood, serum, plasma, saliva, mucus, breath, urine, CSF, sputum, sweat, stool, hair, seminal fluid, biopsy, rhinorrhea, tissue biopsy, cytological sample, platelets, reticulocytes, leukocytes, epithelial cells, or whole blood cells.

In a particular embodiment, the sample is a blood sample - e.g. serum, plasma, whole blood. The sample may be a venous sample, peripheral blood mononuclear cell sample or a peripheral blood sample. Preferably, the sample comprises white blood cells including for example granulocytes, lymphocytes and/or monocytes. In one embodiment, the sample is depleted of red blood cells.

The sample is preferably derived from the subject no more than 72 hours, no more than 60 hours, no more than 48 hours, no more than 36 hours, no more than one 24 hours or even no more than 12 hours following symptom onset.

The sample may be fresh or frozen.

The term "antibody" as used in this invention includes intact molecules as well as functional fragments thereof (such as Fab, F(ab')2, Fv, scFv, dsFv, or single domain molecules such as VH and VL) that are capable of binding to an epitope of an antigen.

Suitable antibody fragments for practicing some embodiments of the invention include a complementarity-determining region (CDR) of an immunoglobulin light chain (referred to herein as "light chain"), a complementarity-determining region of an immunoglobulin heavy chain (referred to herein as "heavy chain"), a variable region of a light chain, a variable region of a heavy chain, a light chain, a heavy chain, an Fdfragment, and antibody fragments comprising essentially whole variable regions of both light and heavy chains such as an Fv, a single chain Fv Fv (scFv), a disulfide-stabilized Fv (dsFv), an Fab, an Fab', and an F(ab')2.

As used herein, the terms "complementarity-determining region" or "CDR" are used interchangeably to refer to the antigen binding regions found within the variable region of the heavy and light chain polypeptides. Generally, antibodies comprise three CDRs in each of the VH (CDR HI or HI; CDR H2 or H2; and CDR H3 or H3) and three in each of the VL (CDR LI or LI; CDR L2 or L2; and CDR L3 or L3).

The identity of the amino acid residues in a particular antibody that make up a variable region or a CDR can be determined using methods well known in the art and include methods such as sequence variability as defined by Kabat et al. (See, e.g., Kabat et al., 1992, Sequences of Proteins of Immunological Interest, 5th ed., Public Health Service, NIH, Washington D.C.), location of the structural loop regions as defined by Chothia et al. (see, e.g., Chothia et al., Nature 342:877-883, 1989.), a compromise between Kabat and Chothia using Oxford Molecular's AbM antibody modeling software (now Accelrys^{®}, see, Martin et al., 1989, Proc. Natl Acad Sci USA. 86:9268; and world wide web site www.bioinf-org.uk/abs), available complex crystal structures as defined by the contact definition (see MacCallum et al., J. Mol. Biol. 262:732-745, 1996) and the "conformational definition" (see, e.g., Makabe et al., Journal of Biological Chemistry, 283:1156-1166, 2008).

As used herein, the "variable regions" and "CDRs" may refer to variable regions and CDRs defined by any approach known in the art, including combinations of approaches.

Functional antibody fragments comprising whole or essentially whole variable regions of both light and heavy chains are defined as follows:
(i) Fv, defined as a genetically engineered fragment consisting of the variable region of the light chain (VL) and the variable region of the heavy chain (VH) expressed as two chains;
(ii) (ii) single chain Fv ("scFv"), a genetically engineered single chain molecule including the variable region of the light chain and the variable region of the heavy chain, linked by a suitable polypeptide linker as a genetically fused single chain molecule.
(iii) (iii) disulfide-stabilized Fv ("dsFv"), a genetically engineered antibody including the variable region of the light chain and the variable region of the heavy chain, linked by a genetically engineered disulfide bond.
(iv) (iv) Fab, a fragment of an antibody molecule containing a monovalent antigen-binding portion of an antibody molecule which can be obtained by treating whole antibody with the enzyme papain to yield the intact light chain and the Fd fragment of the heavy chain which consists of the variable and CH1 domains thereof;
(v) (v) Fab', a fragment of an antibody molecule containing a monovalent antigen-binding portion of an antibody molecule which can be obtained by treating whole antibody with the enzyme pepsin, followed by reduction (two Fab' fragments are obtained per antibody molecule);
(vi) (vi) F(ab')2, a fragment of an antibody molecule containing a monovalent antigen-binding portion of an antibody molecule which can be obtained by treating whole antibody with the enzyme pepsin (i.e., a dimer of Fab' fragments held together by two disulfide bonds); and
(vii) (vii) Single domain antibodies or nanobodies are composed of a single VH or VL domains which exhibit sufficient affinity to the antigen.

Methods of producing polyclonal and monoclonal antibodies as well as fragments thereof are well known in the art (See for example, Harlow and Lane, Antibodies: A Laboratory Manual, Cold Spring Harbor Laboratory, New York, 1988.)

According to a particular embodiment, the antibody is a humanized antibody.

Humanized forms of non-human (e.g., murine) antibodies are chimeric molecules of immunoglobulins, immunoglobulin chains or fragments thereof (such as Fv, Fab, Fab', F(ab').sub.2 or other antigen-binding subsequences of antibodies) which contain minimal sequence derived from non-human immunoglobulin. Humanized antibodies include human immunoglobulins (recipient antibody) in which residues form a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin [Jones et al., Nature, 321:522-525 (1986); Riechmann et al., Nature, 332:323-329 (1988); and Presta, Curr. Op. Struct. Biol., 2:593-596 (1992)].

Methods for humanizing non-human antibodies are well known in the art. Generally, a humanized antibody has one or more amino acid residues introduced into it from a source which is non-human. These non-human amino acid residues are often referred to as import residues, which are typically taken from an import variable domain. Humanization can be essentially performed following the method of Winter and co-workers [Jones et al., Nature, 321:522-525 (1986); Riechmann et al., Nature 332:323-327 (1988); Verhoeyen et al., Science, 239:1534-1536 (1988)], by substituting rodent CDRs or CDR sequences for the corresponding sequences of a human antibody. Accordingly, such humanized antibodies are chimeric antibodies (U.S. Pat. No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries [Hoogenboom and Winter, J. Mol. Biol., 227:381 (1991); Marks et al., J. Mol. Biol., 222:581 (1991)]. The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1):86-95 (1991)]. Similarly, human antibodies can be made by introduction of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Pat. Nos. 5,545,807; 5,545,806; 5,569,825; 5,625,126; 5,633,425; 5,661,016, and in the following scientific publications: Markset al., Bio/Technology 10,: 779-783 (1992); Lonberg et al., Nature 368: 856-859 (1994); Morrison, Nature 368 812-13 (1994); Fishwild et al., Nature Biotechnology 14, 845-51 (1996); Neuberger, Nature Biotechnology 14: 826 (1996); and Lonberg and Huszar, Intern. Rev. Immunol. 13, 65-93 (1995).

### Antibodies used in LFIAs may be polyclonal antibodies

Polyclonal antibodies for measuring any of the polypeptide determinants disclosed herein include without limitation antibodies that were produced from sera by active immunization of one or more of the following: Rabbit, Goat, Sheep, Chicken, Duck, Guinea Pig, Mouse, Donkey, Camel, Rat and Horse.

The antibodies used in the LFIA of the present invention are monoclonal antibodies.

Suitable sources for monoclonal antibodies for the detection of the polypeptides include commercially available sources such as, for example, Abazyme, Abnova, AssayPro, Affinity Biologicals, AntibodyShop, Aviva bioscience, Biogenesis, Biosense Laboratories, Calbiochem, Cell Sciences, Chemicon International, Chemokine, Clontech, Cytolab, DAKO, Diagnostic BioSystems, eBioscience, Endocrine Technologies, Enzo Biochem, Eurogentec, Fusion Antibodies, Genesis Biotech, GloboZymes, Haematologic Technologies, Immunodetect, Immunodiagnostik, Immunometrics, Immunostar, Immunovision, Biogenex, Invitrogen, Jackson ImmunoResearch Laboratory, KMI Diagnostics, Koma Biotech, LabFrontier Life Science Institute, Lee Laboratories, Lifescreen, Maine Biotechnology Services, Mediclone, MicroPharm Ltd., ModiQuest, Molecular Innovations, Molecular Probes, Neoclone, Neuromics, New England Biolabs, Novocastra, Novus Biologicals, Oncogene Research Products, Orbigen, Oxford Biotechnology, Panvera, PerkinElmer Life Sciences, Pharmingen, Phoenix Pharmaceuticals, Pierce Chemical Company, Polymun Scientific, Polysiences, Inc., Promega Corporation, Proteogenix, Protos Immunoresearch, QED Biosciences, Inc., R&D Systems, Repligen, Research Diagnostics, Roboscreen, Santa Cruz Biotechnology, Seikagaku America, Serological Corporation, Serotec, SigmaAldrich, StemCell Technologies, Synaptic Systems GmbH, Technopharm, Terra Nova Biotechnology, TiterMax, Trillium Diagnostics, Upstate Biotechnology, US Biological, Vector Laboratories, Wako Pure Chemical Industries, and Zeptometrix. However, the skilled artisan can routinely make antibodies, against any of the polypeptides described herein.

Monoclonal antibodies for measuring TRAIL include without limitation: Mouse, Monoclonal (55B709-3) IgG; Mouse, Monoclonal (2E5) IgG1; Mouse, Monoclonal (2E05) IgG1; Mouse, Monoclonal (M912292) IgG1 kappa; Mouse, Monoclonal (IIIF6) IgG2b; Mouse, Monoclonal (2E1-1B9) IgG1; Mouse, Monoclonal (RIK-2) IgG1, kappa; Mouse, Monoclonal M181 IgG1; Mouse, Monoclonal VI10E IgG2b; Mouse, Monoclonal MAB375 igG1; Mouse, Monoclonal MAB687 IgG1; Mouse, Monoclonal HS501 IgG1; Mouse, Monoclonal clone 75411.11 Mouse IgG1; Mouse, Monoclonal T8175-50 IgG; Mouse, Monoclonal 2B2.108 IgG1; Mouse, Monoclonal B-T24 IgG1; Mouse, Monoclonal 55B709.3 IgG1; Mouse, Monoclonal D3 IgG1; Goat, Monoclonal C19 IgG; Rabbit, Monoclonal H257 IgG; Mouse, Monoclonal 500-M49 IgG; Mouse, Monoclonal 05-607 IgG; Mouse, Monoclonal B-T24 IgG1; Rat, Monoclonal (N2B2), IgG2a, kappa; Mouse, Monoclonal (1A7-2B7), IgG1; Mouse, Monoclonal (55B709.3), IgG and Mouse, Monoclonal B-S23* IgG1, Human TRAIL/TNFSF10 MAb (Clone 75411), Mouse IgG1, Human TRAIL/TNFSF10 MAb (Clone 124723), Mouse IgG1, Human TRAIL/TNFSF10 MAb (Clone 75402), Mouse lgG1.

Antibodies for measuring TRAIL include monoclonal antibodies and polyclonal antibodies for measuring TRAIL. Antibodies for measuring TRAIL include antibodies that were developed to target epitopes from the list comprising of: Mouse myeloma cell line NS0-derived recombinant human TRAIL (Thr95-Gly281 Accession #P50591), Mouse myeloma cell line, NS0-derived recombinant human TRAIL (Th r95-G ly28 1, with an N-terminal Met and 6-His tag Accession # P50591), E. coli-derived, (Val114-Gly281, with and without an N-terminal Met Accession #:Q6IBA9), Human plasma derived TRAIL, Human serum derived TRAIL, recombinant human TRAIL where first amino acid is between position 85 - 151 and the last amino acid is at position 249 - 281.

Examples of "Monoclonal antibodies for measuring CRP", include without limitation: Mouse, Monoclonal (108-2A2); Mouse, Monoclonal (108-7G41D2); Mouse, Monoclonal (12D-2C-36), IgG1; Mouse, Monoclonal (1G1), IgG1; Mouse, Monoclonal (5A9), IgG2a kappa; Mouse, Monoclonal (63F4), IgG1; Mouse, Monoclonal (67A1), IgG1; Mouse, Monoclonal (8B-5E), IgG1; Mouse, Monoclonal (B893M), IgG2b, lambda; Mouse, Monoclonal (C1), IgG2b; Mouse, Monoclonal (C11F2), IgG; Mouse, Monoclonal (C2), IgG1; Mouse, Monoclonal (C3), IgG1; Mouse, Monoclonal (C4), IgG1; Mouse, Monoclonal (C5), IgG2a; Mouse, Monoclonal (C6), IgG2a; Mouse, Monoclonal (C7), IgG1; Mouse, Monoclonal (CRP103), IgG2b; Mouse, Monoclonal (CRP11), IgG1; Mouse, Monoclonal (CRP135), IgG1; Mouse, Monoclonal (CRP169), IgG2a; Mouse, Monoclonal (CRP30), IgG1; Mouse, Monoclonal (CRP36), IgG2a; Rabbit, Monoclonal (EPR283Y), IgG; Mouse, Monoclonal (KT39), IgG2b; Mouse, Monoclonal (N-a), IgG1; Mouse, Monoclonal (N1G1), IgG1; Monoclonal (P5A9AT); Mouse, Monoclonal (S5G1), IgG1; Mouse, Monoclonal (SB78c), IgG1; Mouse, Monoclonal (SB78d), IgG1 and Rabbit, Monoclonal (Y284), IgG.

Examples of "Monoclonal antibodies for measuring SAA", include without limitation: Mouse, Monoclonal (SAA15), IgG1; Mouse, Monoclonal (504), IgG2b; Mouse, Monoclonal (SAA6), IgG1; Mouse, Monoclonal (585), IgG2b; Mouse, Monoclonal (426), IgG2b; Mouse, Monoclonal (38), IgG2b; Mouse, Monoclonal (132), IgG3; Mouse, Monoclonal (S3-F11), IgM; Mouse, Monoclonal (513), IgG1; Mouse, Monoclonal (291), IgG2b; Mouse, Monoclonal (607), IgG1; Mouse, Monoclonal (115), IgG1; Mouse, Monoclonal (B332A), IgG1; Mouse, Monoclonal (B336A), IgG1; Mouse, Monoclonal (B333A), IgG1; Rabbit, Monoclonal (EPR2927); Rabbit, Monoclonal (EPR4134); Mouse, Monoclonal (Reu86-1), IgG1; Mouse, Monoclonal (Reu86-5), IgG1; Mouse, Monoclonal (291), IgG2b kappa; Mouse, Monoclonal (504), IgG2b kappa; Mouse, Monoclonal (585), IgG2b kappa; Mouse, Monoclonal (S3), IgM kappa; Mouse, Monoclonal (mc1), IgG2a kappa; Mouse, Monoclonal (Reu 86-2), IgG2a; Mouse, Monoclonal (3C11-2C1), IgG2b kappa and Rabbit, Monoclonal (EPR2926), IgG.

Examples of monoclonal antibodies for measuring IP-10 include without limitation: IP-10/CXCL10 Mouse anti-Human Monoclonal (4D5) Antibody (LifeSpan BioSciences), IP-10/CXCL10 Mouse anti-Human Monoclonal (A00163.01) Antibody (LifeSpan BioSciences), MOUSE ANTI HUMAN IP-10 (AbD Serotec), RABBIT ANTI HUMAN IP-10 (AbD Serotec), IP-10 Human mAb 6D4 (Hycult Biotech), Mouse Anti-Human IP-10 Monoclonal Antibody Clone B-C50 (Diaclone), Mouse Anti-Human IP-10 Monoclonal Antibody Clone B-C55 (Diaclone), Human CXCL10/IP-10 MAb Clone 33036 (R&D Systems), CXCL10/INP10 Antibody 1E9 (Novus Biologicals), CXCL10/INP10 Antibody 2C1 (Novus Biologicals), CXCL10/INP10 Antibody 6D4 (Novus Biologicals), CXCL10 monoclonal antibody M01A clone 2C1 (Abnova Corporation), CXCL10 monoclonal antibody (M05), clone 1E9 (Abnova Corporation), CXCL10 monoclonal antibody, clone 1 (Abnova Corporation), IP10 antibody 6D4 (Abcam), IP10 antibody EPR7849 (Abcam), IP10 antibody EPR7850 (Abcam).

Antibodies for measuring IP-10 include monoclonal antibodies for measuring IP-10 and polyclonal antibodies for measuring IP-10.

Antibodies for measuring IP-10 also include antibodies that were developed to target epitopes from the list comprising of: Recombinant human CXCL10/IP-10, non-glycosylated polypeptide chain containing 77 amino acids (aa 22-98) and an N-terminal His tag Interferon gamma inducible protein 10(125 aalong), IP-10 His Tag Human Recombinant IP-10 produced in E. Coli containing 77 amino acids fragment (22-98) and having a total molecular mass of 8.5 kDa with an amino-terminal hexahistidine tag, E. coli-derived Human IP-10 (Val22-Pro98) with an N-terminal Met, Human plasma derived IP-10, Human serum derived IP-10, recombinant human IP-10 where first amino acid is between position 1-24 and the last amino acid is at position 71-98.

The immunoassay performed by the LFI device of the present invention may be a competitive or non-competitive binding immunoassay, the principles of which are well known to those skilled in the art and are further described herein below.

In one embodiment, the immunoassay comprises a competitive binding assay, where the labeled antibody but not the immunocomplex forms a visible signal in the test band. If a sufficient amount of TRAIL is present in the sample, there will remain none or only an insubstantial amount of unbound labeled antibody. Thus, if a sample contains sufficient TRAIL, the labeled antibody will not bind to the TRAIL antibody capture reagent at the test band, and therefore will not be detectable in the test band. This result will be referred to herein as a positive result, indicating that it is positive for a sufficient amount of TRAIL in the sample.

In an alternative embodiment, the immunocomplex of TRAIL + labeled antibody, but not the non-complexed labeled antibody forms a visible signal in the test band. Therefore, if a sufficient amount of TRAIL is present in the sample, a sufficient amount of immunocomplex will form and will be detectable in the test band. This result is also a positive result, indicating that it is positive for a sufficient amount of TRAIL in the sample.

An exemplary device is illustrated in Figure 2A. The device (10) comprises an immunoassay test strip (20), which comprises a sample pad (12), a conjugate pad (14), a test area (16), an absorbent pad (also referred to as a wick (18), and a backing (22). In one embodiment, the conjugate pad comprises mobile labeled antibody while the test area (16) comprises an immobilized antigen (e.g. TRAIL polypeptide). The labeled antibody will bind to the immobilized antigen, unless it is blocked by antigen (e.g. TRAIL polypeptide) present in the sample.

In another embodiment, the conjugate pad comprises mobile labeled antibody while the test area (16) comprises a non-labeled antibody that recognizes the same determinant as the labeled antibody (e.g. TRAIL polypeptide). The labeled complex will bind to the immobilized antibody at the test area (16). The present inventors contemplate various combinations of antibodies in the assay which are summarized below:
1. the mobile labeled antibody is monoclonal, and the immobilized (non-labeled antibody) is monoclonal;
2. the mobile labeled antibody is monoclonal, and the immobilized (non-labeled antibody) is polyclonal;
3. the mobile labeled antibody is polyclonal, and the immobilized (non-labeled antibody) is monoclonal;
4. the mobile labeled antibody is polyclonal, and the immobilized (non-labeled antibody) is polyclonal;

The order of events in a LFIA is typically as follows:
The sample pad (12) receives the sample and may be comprised of a fibrous material which absorbs the sample. The sample pad (12) may be formed of cotton, glass fiber, rayon, polyester, nylon, cellulose, spun polyethylene, or other suitable materials.

The sample is then drawn into the conjugate pad (14) which releases a labeled antibody into the sample. The conjugate pad (14) may be formed of polyesters, rayons or glass fibers.

The antibody at the conjugate pad (14) is conjugated to a detectable label which provides a means of visualizing or detecting the antibody-antigen complex, and may comprise a chromogen, catalyst, fluorescent compound, chemiluminescent compound, colloidal gold, a dye particle, a latex particle tagged with a detector reagent such as, for example, a colored or fluorescent dye, and the like.

Labels are further described herein below.

After crossing the conjugate pad, the sample then passes into the test area (16), which comprises a test band (26), and may also comprise a control band (24). The test area may be a membrane which may be comprised of nitrocellulose or a similar blotting material. The test and control bands (24, 26) each comprise a suitable capture reagent which has been immobilized in a particular area of the test area (16) to "capture" or bind a specific molecule. As used herein, the term "immobilized" means the capture reagent is attached to or confined within the control band (24) or test band (26) such that lateral flow of fluids across the test strip (20) during the immunoassay will not dislodge the capture reagent. In one embodiment, the control band (24) is positioned downstream of the test band (26).

The sample and dilution buffer liquid moves past the control and test bands (24, 26) and collects in the wick (18) which prevents the backflow of the fluid into the device (10) or accidental leakage of the fluid following testing.

In an embodiment of a competitive assay, a first capture reagent immobilized in the test band (26) comprises TRAIL polypeptide. Accordingly, any labeled antibody which is not part of an immunocomplex will bind to the first capture reagent. The first capture reagent may be immobilized in the test band by conjugating it to a bulky protein. Suitable proteins have a size which is larger than the pore size of the detection membrane, and may include, but are not limited to, bovine serum albumin, keyhole limpet hemocyanin, thryoglobulin, and ovalbumin. In one embodiment, the protein is selected from bovine serum albumin or keyhole limpet hemocyanin. The bulky protein prevents migration of the first capture reagent through the detection membrane.

If present in sufficient quantity, TRAIL within the sample interacts with the labeled antibody to form a complex, leaving no unbound labeled antibody (or an insignificant amount). The complex then migrates from the conjugate pad (14) to the test area (16). The labeled antibody, which is already bound to the analyte (i.e. TRAIL), subsequently does not bind to the capture reagent immobilized at the test band (26). The absence of a detectable colored stripe at the location of the test band (26) indicates the presence of the analyte (e.g. TRAIL) in the sample (i.e., a positive result). This result indicates that the TRAIL level is above a predetermined level e.g. more than 100 pg/ml, more than 120 pg/ml or more than 150 pg/ml.

If there is insufficient TRAIL to bind all or substantially all of the labeled antibody, the unbound labeled antibody will form a detectable signal in the test band. This result indicates that the TRAIL level is below a predetermined level e.g. less than 75 pg/ml, less than 50 pg/ml or less than 25 pg/ml.

In an alternative embodiment, a non-competitive immunoassay may comprise a sandwich assay in which the antigen/antibody complex, if present, also binds to an antibody specific to the TRAIL antibody which is fixed in the test band. The antibody at the test band does not comprise the same label as that which is at the conjugate pad. In a particular embodiment, the antibody at the test band is not labeled. Thus, the first capture reagent may comprise another antibody specific to TRAIL. As a result, the immunocomplex, but not unbound labeled antibody will bind to the test band and be detectable. In this case, a positive result will be indicated by the presence of a detectable signal in the test band.

It will be appreciated that the test area (16) may comprise more than one test band (26). The additional test band may detect the same analyte as in the first test band (e.g. TRAIL antibody; see for example Figures 12A-B) or may detect an additional determinant.

Additional determinants that may be analyzed are summarized herein below. It will be appreciated that all the configurations described herein above for TRAIL are appropriate for detecting such additional determinants (using the corresponding antibodies).

In one embodiment, the test area (16) comprises two test bands (26), a firsttest band which indicates the level of TRAIL, a second test band which indicates the level of CRP. Figures 4A-B illustrates the order of the contemplated test bands according to embodiments of this aspect of the present invention.

In another embodiment, the test area (16) comprises two test bands (26), a first test band which indicates the level of TRAIL, a second test band which indicates the level of IP10.

In one embodiment, the test area (16) comprises three test bands (26), a first test band which indicates the level of TRAIL, a second test band which indicates the level of CRP and a third test band which indicates the level of IP-10. Figures 1A-F illustrate the order of the contemplated test bands according to embodiments of this aspect of the present invention.

In Figure 1A, the test band for TRAIL (26A) appears first, the test band for CRP (26B) appears second and the test band for IP10 (26C) appears third, with the order being according to the lateral flow.

In Figure 1B, the test band for TRAIL (26A) appears first, the test band for CRP (26B) appears third and the test band for IP10 (26C) appears second, with the order being according to the lateral flow.

In Figure 1C, the test band for IP10 (26C) appears first, the test band for TRAIL (26A) appears second and the test band for CRP (26B) appears third, with the order being according to the lateral flow.

In Figure 10, the test band for IP10 (26C) appears first, the test band for TRAIL (26A) appears third and the test band for CRP (26B) appears second, with the order being according to the lateral flow.

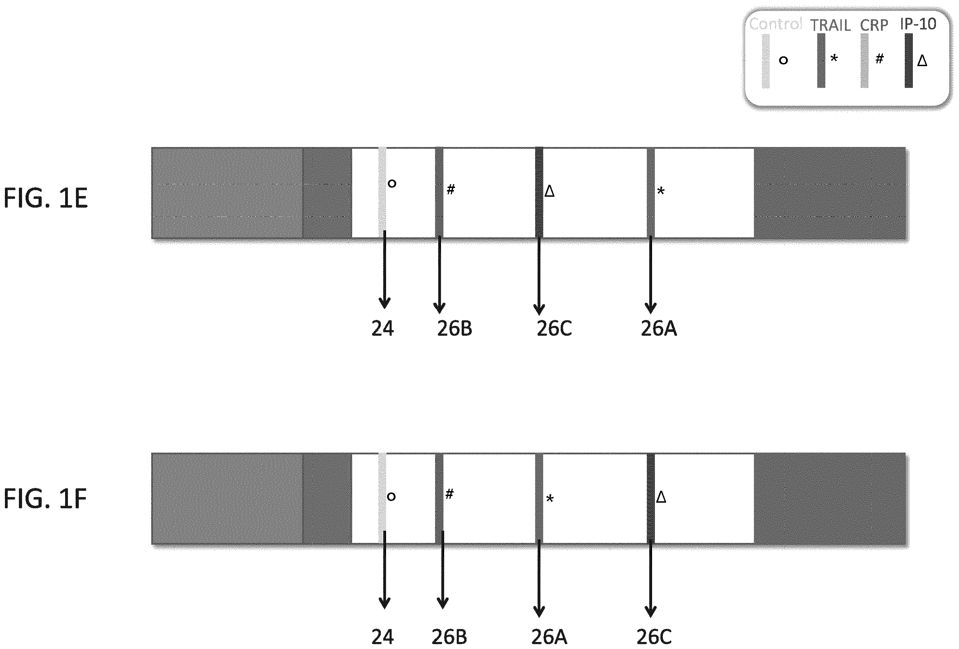

In Figure 1E, the test band for CRP (26B) appears first, the test band for IP10 (26C) appears second, and the test band for TRAIL (26A) appears third, with the order being according to the lateral flow.

In Figure 1F, the test band for CRP (26B) appears first, the test band for IP10 (26C) appears third, and the test band for TRAIL (26A) appears second, with the order being according to the lateral flow.

The control band (24) comprises a capture reagent which will bind to the labeled antibody regardless of whether or not it has bound to the antigen TRAIL. For example, the control band capture reagent may comprise an immobilized antibody specific to the antibody portion of the antigen/antibody complex. The control band (24) may be placedbefore the test band (26) as illustrated in Figures 1A-F or after the test band (as illustrated in Figure 2A).

In another embodiment, the strength or intensity of the detectable signal may vary along a scale or gradient, and be related to the concentration of analyte in the sample. In this case, the immunoassay may be quantitative rather than qualitative.

One embodiment of a quantitative or semi-quantitative assay is depicted in Figures 12A-B for TRAIL. In this embodiment, more than one test band (26A) for the same determinant is present on the test strip (20). The capture reagent at each of the test bands may be present at the same concentration or at increasing concentrations.

In one embodiment, a detectable "control" signal forms at the control band (24) irrespective of the result at the test band (26). The control band indicates that the sample has flowed through the test strip (20) and that the test is valid and functioning properly. The control band is preferably downstream from the test band.

When the test strip is used, a fluid sample is added to the sample pad (14) for example, by pipette or medicine dropper. In one embodiment, the amount of the fluid sample ranges from about 1 µL to about 100 µL. In one embodiment, the amount of the fluid sample is about 20 µ.L. The sample may be pre-treated to facilitate the release of TRAIL from its receptor in the sample. In one embodiment, the sample is treated with urea, guanidine hydrochloride, an acidic solution, or an alkaline solution. The sample migrates via capillary action from the sample pad (12) to the conjugate pad (14). The labeled antibody is mobilized by the movement of fluid through the conjugate pad (14), and is carried by the flow through the test area (16). Dilution buffer may be added to provide sufficient testing volume, and to ensure optimum lateral capillary flow as the sample migrates through the entire length of the test strip (20). In one embodiment, dilution buffer is added separately from the sample, and may be added upstream from the sample pad.

The wick (18) is downstream from the detection membrane of the test area (16), and serves to "pull" the fluids added to the test strip (20) for the duration of the immunoassay by absorbing the fluids. Preferably, the wick (18) is of sufficient capacity and absorption ability to ensure that fluids do not backflow into the detection membrane of the test area (16), which may compromise the test results. The wick (18) is formed of a hydrophilic material, such as high-density cellulose, glass fibre/cellulose mix, cotton linter or other suitable materials.

The backing (22) serves as a physical support or base upon which the sample pad (12), the conjugate pad (14), the test area (16), and the wick (18) are mounted. In one embodiment, the backing (22) is formed of a plastic material strip such as, for example, polystyrene. The test components (12, 14, 16, 18) may be mounted to the backing (22) by an adhesive. The components (12, 14, 16, 18) are mounted so as to abut each other, or overlap onto one another to maintain the flow of fluids across the test strip (20).

The test strip (20) and its components may be fabricated using techniques known to those skilled in the art. The conjugate pad (14) is pre-treated with labeled antibody by dispensing or dipping, followed by drying. The capture reagents at the control and test bands (24, 26) can be immobilized using several methods well known to those skilled in the art including, for example, direct adsorption and covalent attachment. Blocking of non-specific binding may be achieved by coating the surface of the tests area (16) with blocking buffers such as for example, bovine serum albumin, followed by drying. The sample pad (12) may also be pre-treated to filter out particulates, bind sample components which might interfere with the immunoassay, or disrupt the sample to release the target analyte. The components (12, 14, 16, 18 and 22) may be assembled into cards, with the sample pad (12), conjugate pad (14), test area (16), and wick (18) being mounted onto the backing (22) using an appropriate adhesive. The cards may then be cut into individual strips.

The present invention contemplates using more than one test strip (20) per assay.

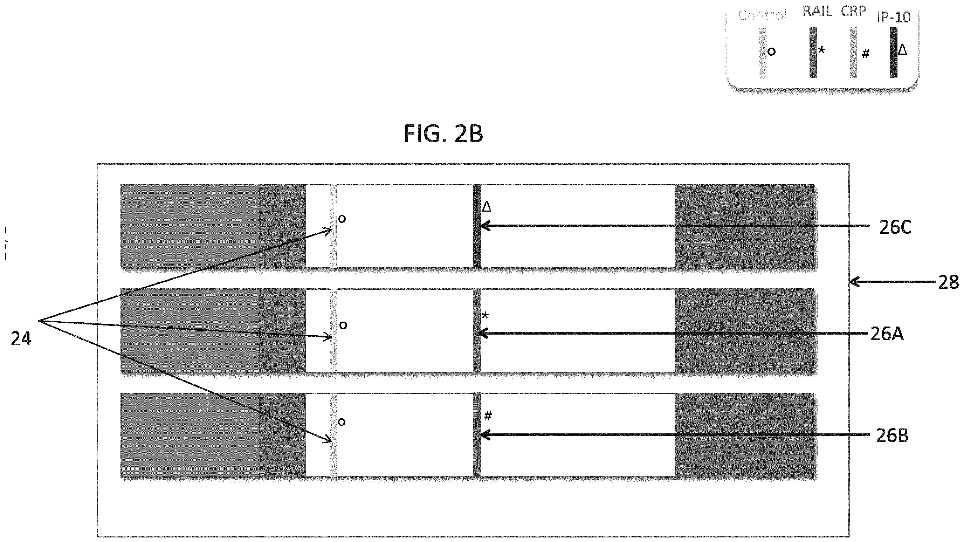

Figures 2B-G represent an embodiment, wherein one assay uses three test strips (20), wherein each test strip comprises a single test band (26A, B or C) and a single control band (24). Different contemplated orders of the strips in the cassette (28) are shown.

Figures 3A-F represent an embodiment, wherein one assay uses two test strips (20), wherein one test strip comprises a single test band (26A, B or C) and a single control band (24) and the other test strip comprises two test bands (from 26A, B and/or C) and a single control band (24). Different contemplated orders of the strips in the cassette (28) are shown.

Figure 5 represent an embodiment, wherein one assay uses two test strips (20), wherein one test strip comprises a single test band (26A- for TRAIL) and a single control band (24) and the other test strip comprises a single test band (26B - for CRP) and a single control band.

In one embodiment, the test strip (20) may be used directly or housed within a cassette (28) to facilitate handling. The cassette (28) comprises a housing and defines a sample aperture for introducing the sample into the immunoassay, with the sample pad (12) positioned beneath the sample aperture. In one embodiment, the cassette (28) defines a buffer aperture for introducing dilution buffer into the immunoassay. The cassette may comprises a test window positioned above the control and test bands (24, 26) of the test area (16), to permit visualization or detection of the control and test bands. The test window is preferably formed of a transparent polymer material. Test results may thus be viewed through the test window by eye, a detector, or reader system. Non-limiting examples of detection systems include spectrophotometers, reflectance readers, luminometers, fluorometers, photodetectors or photomultipliertubes, scintillation counters, and other suitable instruments. Detection systems are further described herein below.

The test strips or device may be in the form of a kit which includes necessary antibodies, antigens, or buffered diluents as separate reagents, or in the form of a cassette comprising all needed antibodies and antigens. A sample collector such as a finger prick needle may be included.

Examples of buffers and/or washes that may be added to the kit (in order to reduce non-specific binding and increase signal to noise ratio) include high salt buffers (to exclude any non-specific binding due to ionic bonds), for example TRIS-NaCL, PBS, RIPA, HEPES-NaCL etc., detergents (to exclude any non-specific binding due to Van-Der-Vales bonds) as Dodecyl sodium sulfate ( SDS), Triton-X-100, Triton-X-14, Tween-20, Tween-80, Brij, Digitonin, Cholic acid, Sodium taurodeoxycholate, CHAPSO and the same, anti-foam formulation and a preservative (a preservative can be Proclin 300, sodium azide, Thimerosal, 2-Methylisothiazol-3(2H)-one, 2-BROMO-2-NITROPROPANE-1,3-DIL.

***Detection systems:*** In one embodiment, the detectable signal is observed in about ten minutes or less.

In another embodiment, the detectable signal is observed in about 5 minutes or less.

In still another embodiment, the detectable signal is observed no longer than 3 minutes.

In case of gold nanoparticles or other color producing labels, qualitative or semi-quantitative analysis can be done by visual inspection of colors at test and control lines. The major advantage of visual inspection is rapid qualitative answer in "Yes" or "NO". Such quick replies about presence of an analyte in clinical analysis have very high importance. Such tests help doctors to make an immediate decision near the patients in hospitals in situations where test results from central labs cannot be waited for because of huge time consumption. But for quantification, optical strip readers are employed for measurement of the intensity of colors produced at test and control lines of strip. This is achieved by inserting the strips into a strip reader and intensities are recorded simultaneously by imaging softwares. Optical images of the strips can also be recorded with a camera and then processed by using a suitable software. Procedure includes proper placement of strip under the camera and a controlled amount of light is thrown on the areas to be observed. Such systems use monochromatic light and wavelength of light can be adjusted to get a good contrast among test and control lines and background. In orderto provide good quantitative and reproducible results, detection system should be sensitive to different intensities of colors. Optical standards can be used to calibrate an optical reader device. Automated systems have advantages over manual imaging and processing in terms of time consumption, interpretation of results and adjustment of variables.

In case of fluorescent labels, a fluorescence strip reader is used to record fluorescence intensity of test and control lines. Fluorescence brightness of test line increased with an increase in nitrated seruloplasmin concentration in human serum when it was detected with a fluorescence strip reader. A photoelectric sensor was also used for detection in LFIA where colloidal gold is exposed to light emitting diode and resulting photoelectrons are recorded. Chemiluminescence which results from reaction of enzyme and substrate is measured as a response to amount of target analyte. Magnetic strip readers and electrochemical detectors are also reported as detection systems in LFTS but they are not very common. Selection of detector is mainly determined by the label employed in analysis.

### Interpretation of results

The immunoassay may be a qualitative test, providing a "yes" or "no" result in the form of a detectable signal, such as a color change or difference on the test strip. In one embodiment, the immunoassay of the present invention comprises a competitive inhibition binding test. In such a test, a molecule competes with another molecule for binding to the same target. In one embodiment, if the sample contains a sufficient amount of the analyte, a positive result is indicated by the absence of a detectable colored stripe at the test band (26). A negative result is indicated by the presence of a detectable colored stripe at the test band (26).

The threshold value which divides positive and negative results may be determined by the concentration or quantity of the labeled antibody and the first capture reagent. The immunoassay may be made more sensitive to lower concentrations of TRAIL by having a reduced quantity or concentration of the labeled antibody. Conversely, its sensitivity may be decreased by increasing the quantity or concentration of the labeled antibody, in which case a greater quantity of TRAIL will be required to block all the labeled antibody.

In one embodiment, the LIFA is used as a point of care device, where the user interprets the results directly. In another embodiment, the results are delivered to an laboratory information system (LIS) or to a cloud based database.

The level of TRAIL may be used to distinguish between an infective or non-infective state.

The level of TRAIL may be used to distinguish between a bacterial and viral infection. The level of TRAIL may be used to distinguish between a bacterial and bacterial/viral co-infection.

The level of TRAIL may be used to distinguish between a viral and bacterial/viral co-infection.

Thus, the level of TRAIL can be used to rule in a bacterial infection, rule in a viral infection, rule in a bacterial/viral infection or rule in a non-infectious state. Once a bacterial infection or mixed bacterial/viral infection has been ruled in, the subject may be treated with an antibiotic.

Examples of antibiotic agents include, but are not limited to Daptomycin; Gemifloxacin; Telavancin; Ceftaroline; Fidaxomicin; Amoxicillin; Ampicillin; Bacampicillin; Carbenicillin; Cloxacillin; Dicloxacillin; Flucloxacillin; Mezlocillin; Nafcillin; Oxacillin; Penicillin G; Penicillin V; Piperacillin; Pivampicillin; Pivmecillinam; Ticarcillin; Aztreonam; Imipenem; Doripenem; Meropenem; Ertapenem; Clindamycin; Lincomycin; Pristinamycin; Quinupristin; Cefacetrile (cephacetrile); Cefadroxil (cefadroxyl); Cefalexin (cephalexin); Cefaloglycin (cephaloglycin); Cefalonium (cephalonium); Cefaloridine (cephaloradine); Cefalotin (cephalothin); Cefapirin (cephapirin); Cefatrizine; Cefazaflur; Cefazedone; Cefazolin (cephazolin);Cefradine (cephradine); Cefroxadine; Ceftezole; Cefaclor; Cefamandole; Cefmetazole; Cefonicid; Cefotetan; Cefoxitin; Cefprozil (cefproxil); Cefuroxime; Cefuzonam; Cefcapene; Cefdaloxime; Cefdinir; Cefditoren; Cefetamet; Cefixime; Cefmenoxime; Cefodizime; Cefotaxime; Cefpimizole; Cefpodoxime; Cefteram; Ceftibuten; Ceftiofur; Ceftiolene; Ceftizoxime; Ceftriaxone; Cefoperazone; Ceftazidime; Cefclidine; Cefepime; Cefluprenam; Cefoselis; Cefozopran; Cefpirome; Cefquinome; Fifth Generation; Ceftobiprole; Ceftaroline; NotClassified; Cefaclomezine; Cefaloram; Cefaparole; Cefcanel; Cefedrolor; Cefempidone; Cefetrizole; Cefivitril; Cefmatilen; Cefmepidium; Cefovecin; Cefoxazole; Cefrotil; Cefsumide; Cefuracetime; Ceftioxide; Azithromycin; Erythromycin; Clarithromycin; Dirithromycin; Roxithromycin; Telithromycin; Amikacin; Gentamicin; Kanamycin; Neomycin; Netilmicin; Paromomycin; Streptomycin; Tobramycin; Flumequine; Nalidixic acid; Oxolinic acid; Piromidic acid; Pipemidic acid; Rosoxacin; Ciprofloxacin; Enoxacin; Lomefloxacin; Nadifloxacin; Norfloxacin; Ofloxacin; Pefloxacin; Rufloxacin; Balofloxacin; Gatifloxacin; Grepafloxacin; Levofloxacin; Moxifloxacin; Pazufloxacin; Sparfloxacin; Temafloxacin; Tosufloxacin; Besifloxacin; Clinafloxacin; Gemifloxacin; Sitafloxacin; Trovafloxacin; Prulifloxacin; Sulfamethizole; Sulfamethoxazole; Sulfisoxazole; Trimethoprim-Sulfamethoxazole; Demeclocycline; Doxycycline; Minocycline; Oxytetracycline; Tetracycline; Tigecycline; Chloramphenicol; Metronidazole; Tinidazole; Nitrofurantoin; Vancomycin; Teicoplanin; Telavancin; Linezolid; Cycloserine 2; Rifampin; Rifabutin; Rifapentine; Bacitracin; Polymyxin B; Viomycin; Capreomycin.

Once a viral infection has been ruled in, the subject may be treated with an anti-viral treatment.

An "anti-viral treatment" includes the administration of a compound, drug, regimen or an action that when performed by a subject with a viral infection can contribute to the subject's recovery from the infection or to a relief from symptoms. Examples of antiviral agents include, but are not limited to Abacavir; Aciclovir; Acyclovir; Adefovir; Amantadine; Amprenavir; Ampligen; Arbidol; Atazanavir; Atripla; Balavir; Boceprevirertet; Cidofovir; Combivir; Dolutegravir; Darunavir; Delavirdine; Didanosine; Docosanol; Edoxudine; Efavirenz; Emtricitabine; Enfuvirtide; Entecavir; Ecoliever; Famciclovir; Fomivirsen; Fosamprenavir; Foscarnet; Fosfonet; Fusion inhibitor; Ganciclovir; Ibacitabine; Imunovir; Idoxuridine; Imiquimod; Indinavir; Inosine; Integrase inhibitor; Interferon type III; Interferon type II; Interferon type I; Interferon; Lamivudine; Lopinavir; Loviride; Maraviroc; Moroxydine; Methisazone; Nelfinavir; Nevirapine; Nexavir; Oseltamivir; Peginterferon alfa-2a; Penciclovir; Peramivir; Pleconaril; Podophyllotoxin; Raltegravir; Reverse transcriptase inhibitor; Ribavirin; Rimantadine; Ritonavir; Pyramidine; Saquinavir; Sofosbuvir; StavudineTelaprevir; Tenofovir; Tenofovir disoproxil; Tipranavir; Trifluridine; Trizivir; Tromantadine; Truvada; traporved; Valaciclovir; Valganciclovir; Vicriviroc; Vidarabine; Viramidine; Zalcitabine; Zanamivir; Zidovudine; RNAi antivirals; inhaled rhibovirons; monoclonal antibody respigams; neuriminidase blocking agents.

The level of TRAIL may be used to rule in an acute infection (e.g. an acute bacterial infection).

An "Acute Infection" is characterized by rapid onset of disease, a relatively brief period of symptoms, and resolution within days.

The level of TRAIL may be used to rule in a chronic infection.

A "chronic infection" is an infection that develops slowly and lasts a long time. Viruses that may cause a chronic infection include Hepatitis C and HIV. One difference between acute and chronic infection is that during acute infection the immune system often produces IgM+ antibodies against the infectious agent, whereas the chronic phase of the infection is usually characteristic of IgM-/IgG+ antibodies. In addition, acute infections cause immune mediated necrotic processes while chronic infections often cause inflammatory mediated fibrotic processes and scaring (e.g. Hepatitis C in the liver). Thus, acute and chronic infections may elicit different underlying immunological mechanisms.

By infection type is meantto include bacterial infections, mixed infections, viral infections, no infection, infectious or non-infectious.

By "ruling in" an infection it is meant that the subject has that type of infection.

Furthermore, the level of TRAIL can be used to rule out a bacterial infection, rule out a viral infection, rule out a bacterial/viral infection or rule out a non-infectious state.

By "ruling out" an infection it is meant that the subject does not have that type of infection.

A threshold value for TRAIL which may be used to indicate a bacterial infection may be less than 100 pg/ml, less than 90 pg/ml, less than 80 pg/ml, less than 70 pg/ml, less than 60 pg/ml, less than 50 pg/ml, less than 40 pg/ml.

A threshold value for TRAIL which may be used to indicate a viral infection may be greater than 100 pg/ml, greater than 110 pg/ml, greater than 120 pg/ml, greater than 130 pg/ml, greater than 140 pg/ml, less than 150 pg/ml, greater than 160 pg/ml.

In one embodiment, the immunoassay is configured so that TRAIL can be classified as being low (<70 pg/ml), medium (70-100 pg/ml) or high (>100 pg/ml).

The immunoassay may also be configured so that CRP can be classified as being low (<20 µg/ml), medium (20-80 µg/ml) or high (>80 µg/ml). Optionally, IP10 may also be detected using the same assay. In this case, the immunoassay may be configured so that IP10 can be classified as being low (<200 pg/ml), medium (200-500 pg/ml) or high (>500 pg/ml).

Figures 6-9 provide exemplary readouts when the assay is configured according to particular embodiments. It will be appreciated that these figures are for illustration purposes only, since in real-life, whenever a "high" band is observed, typically a "medium" band and a "low" band will be observed as well.

Thus, for example, Figure 6Ashows an exemplary readout when TRAIL is classified as "high"; CRP is classified as low; and IP10 is classified as high. This readout may indicate a viral infection.

Figure 6B shows an exemplary readout when TRAIL is classified as "medium"; CRP is classified as low; and IP10 is classified as high. This readout may indicate a viral infection.

Figure 6C shows an exemplary readout when TRAIL is classified as "high"; CRP is classified as medium; and IP10 is classified as high. This readout may indicate a viral infection.

Figure 6D shows an exemplary readout when TRAIL is classified as "high"; CRP is classified as low; and IP10 is classified as medium. This readout may indicate a viral infection.

Figure 7A shows an exemplary readout when TRAIL is classified as "medium"; CRP is classified as high; and IP10 is classified as medium. This readout may indicate a bacterial infection.

Figure 7B shows an exemplary readout when TRAIL is classified as "low"; CRP is classified as medium; and IP10 is classified as medium. This readout may indicate a bacterial infection.

Figure 7C shows an exemplary readout when TRAIL is classified as "low"; CRP is classified as high; and IP10 is classified as medium. This readout may indicate a bacterial infection.

Figure 7D shows an exemplary readout when TRAIL is classified as "medium"; CRP is classified as medium; and IP10 is classified as medium. This readout may indicate a bacterial infection.

Figure 8 shows an exemplary readout when TRAIL is classified as "high"; CRP is classified as high; and IP10 is classified as high. This readout may indicate a bacterial/viral co-infection.

Figure 9A shows an exemplary readout when TRAIL is classified as "medium"; CRP is classified as low; and IP10 is classified as medium. This readout may indicate a non-infectious state.

Figure 9B shows an exemplary readout when TRAIL is classified as "medium"; CRP is classified as low; and IP10 is classified as low. This readout may indicate a non-infectious state.

In another embodiment, the immunoassay is configured so that TRAIL can be classified as being low (e.g. <70 pg/ml) or high (e.g. > 70 pg/ml).

The immunoassay may also be configured so that CRP can be classified as being low (e.g. <40 µg/ml) or high (e.g. >40 µg/ml). Optionally, IP10 may also be detected using the same assay. In this case, the immunoassay may be configured so that IP10 can be classified as being low (e.g. <300 pg/ml) or high (e.g. >300 pg/ml).

Figures 10A-D provide exemplary readouts when the assay is configured according to such embodiments.

Thus, for example, Figure 10A shows an exemplary readout when TRAIL is classified as "high"; CRP is classified as low; and IP10 is classified as high. This readout may indicate a viral infection.

Figure 10A shows an exemplary readout when TRAIL is classified as "high"; CRP is classified as low; and IP10 is classified as high. This readout may indicate a viral infection.

Figure 10B shows an exemplary readout when TRAIL is classified as "low"; CRP is classified as high; and IP10 is classified as low. This readout may indicate a bacterial infection.

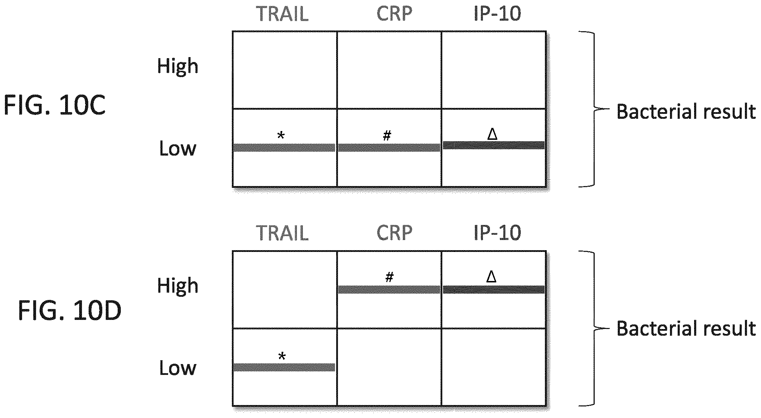

Figure 10C shows an exemplary readout when TRAIL is classified as "low"; CRP is classified as low; and IP10 is classified as low. This readout may indicate a bacterial infection.

Figure 10D shows an exemplary readout when TRAIL is classified as "low"; CRP is classified as high; and IP10 is classified as high. This readout may indicate a bacterial infection.

In another embodiment, the immunoassay is configured so that TRAIL can be classified as being low (e.g. <70 pg/ml), medium (e.g. 70-100 pg/ml) or high e.g. (e.g. >100 pg/ml)). The immunoassay may also be configured so that CRP can be classified as being low (e.g. <20 µg/ml), medium (e.g. 20-80 µg/ml) or high (e.g. >80 µg/ml).

Figures 11A-C provide exemplary readouts when the assay is configured according to particular embodiments.

Thus, for example, Figure 11A shows an exemplary readout when TRAIL is classified as "high"; CRP is classified as low, this readout may indicate a viral infection. When TRAIL is classified as "high"; CRP is classified as medium, this readout may indicate a viral infection. When TRAIL is classified as "medium"; CRP is classified as low, this readout may indicate a viral infection.

Figure 11B shows an exemplary readout when TRAIL is classified as "low"; CRP is classified as low, this readout may indicate a bacterial infection. When TRAIL is classified as "low"; CRP is classified as medium, this readout may indicate a bacterial infection. When TRAIL is classified as "low"; CRP is classified as high, this readout may indicate a bacterial infection. When TRAIL is classified as "medium"; CRP is classified as high, this readout may indicate a bacterial infection.

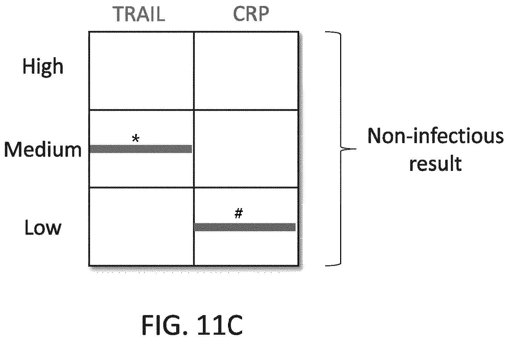

Figure 11C shows an exemplary readout when TRAIL is classified as "medium"; CRP is classified as low; this readout may indicate a non-infectious state.

Classification of subjects into subgroups (e.g. bacterially infected/viral infected; infectious/non-infectious) according to this aspect of the present invention is preferably done with an acceptable level of clinical or diagnostic accuracy. An "acceptable degree of diagnostic accuracy", is herein defined as a test or assay (such as the test used in some aspects of the invention) in which the AUC (area under the ROC curve for the test or assay) is at least 0.60, desirably at least 0.65, more desirably at least 0.70, preferably at least 0.75, more preferably at least 0.80, and most preferably at least 0.85.

By a "very high degree of diagnostic accuracy", it is meant a test or assay in which the AUC (area under the ROC curve for the test or assay) is at least 0.75, 0.80, desirably at least 0.85, more desirably at least 0.875, preferably at least 0.90, more preferably at least 0.925, and most preferably at least 0.95.

Alternatively, the methods predict risk with at least 75% total accuracy, more preferably 80%, 85%, 90%, 95%, 97%, 98%, 99% or greater total accuracy. Alternatively, the methods predict the correct management or treatment with an MCC larger than 0.2, 0.3, 0.4, 0.5, 0.6, 0.7, 0.8, 0.9 or 1.0.

It will be appreciated that the TRAIL level may be used in conjunction with other markers/tests/disease associated parameters etc. in order to diagnose the patient. According to the claimed invention, the amount of TRAIL and CRP polypeptide is used to diagnose the subject with a bacterial or viral infection.

Furthermore, traditional risk factors and additional clinical parameters may be used to classify the severity of the disease.

"Traditional laboratory risk factors" encompass biomarkers isolated or derived from subject samples and which are currently evaluated in the clinical laboratory and used in traditional global risk assessment algorithms, such as absolute neutrophil count (abbreviated ANC), absolute lymphocyte count (abbreviated ALC), white blood count (abbreviated WBC), neutrophil % (defined as the fraction of white blood cells that are neutrophils and abbreviated Neu (%)), lymphocyte % (defined as the fraction of white blood cells that are lymphocytes and abbreviated Lym (%)), monocyte % (defined as the fraction of white blood cells that are monocytes and abbreviated Mon (%)),Sodium (abbreviated Na), Potassium (abbreviated K), Bilirubin (abbreviated Bili).

"Clinical parameters" encompass all non-sample or non-analyte biomarkers of subject health status or other characteristics, such as, without limitation, age (Age), ethnicity (RACE), gender (Sex), core body temperature (abbreviated "temperature"), maximal core body temperature since initial appearance of symptoms (abbreviated "maximal temperature"), time from initial appearance of symptoms (abbreviated "time from symptoms") or family history (abbreviated FamHX).

Determining the severity of a disease may be effected using TRAIL alone or together with a disease associated parameter. The phrase "disease associated parameter" includes biomarkers or determinants such as polypeptides, polynucleotides and metabolites as well as clinical determinants.

Examples of clinical determinants include but are not limited to ANC, ALC, Neu (%), Lym (%), Mono (%), Maximal temperature, time from symptoms, heart rate, blood pressure, age, Creatinine (Cr), Potassium (K), Pulse and Urea.

Other exemplary blood biomarkers which may be measured according to this aspect of the present invention include but are not limited to creatin, serum albumin, and interleukin-6.

Additional combinations of markers which may be used for risk management, treatment course and/or distinguishing between viral and bacterial infections include but are not limited to:
TRAIL+CRP; TRAIL+CRP+IP10; TRAIL+PCT; TRAIL+IL-6; TRAIL+IP-10; TRAIL+NGAL; TRAIL+CRP+PCT; TRAIL+CRP+NGAL; TRAIL+CRP+IP-10; TRAIL+CRP+IL-6; TRAIL+PCT+IL-6; TRAIL+PCT+IP-10; TRAIL+PCT+NGAL; TRAIL+CRP+IL-6+PCT; TRAIL+CRP+IL-6+NGAL; TRAIL+CRP+IL-6+IP-10; TRAIL+NGAL+IL-6+PCT; TRAIL+IP-10+IL-6+PCT; TRAIL + neopterin; TRAIL+WBC; TRAIL+ANC; TRAIL+temperature; TRAIL+mean arterial pressure; TRAIL+pH arterial; TRAIL+heart rate; TRAIL+respiratory rate; TRAIL+AaDO2 or PaO2; TRAIL+sodium; TRAIL+potassium; TRAIL+creatinine; TRAIL+hematocrit; TRAIL+MX1+CRP;TRAIL+MX1+RSAD2; TRAIL+CRP+RSAD2.

### Labeling

As mentioned herein above, the antibody which is immobilized at the conjugate pad is labeled.

Any material that is used as a label should be detectable at very low concentrations and it should retain its properties upon conjugation with biorecognition molecules. This conjugation is also expected not to change features of biorecognition probes. Ease in conjugation with biomolecules and stability over longer period of time are desirable features for a good label. Concentrations of labels down to 10⁻⁹ M are optically detectable. After the completion of assay, some labels generate direct signal (as color from gold colloidal) while others require additional steps to produce analytical signal (as enzymes produce detectable product upon reaction with suitable substrate). Hence the labels which give direct signal are preferable in LFA because of less time consumption and reduced procedure.

***Gold nanoparticles:*** Colloidal gold nanoparticles are the most commonly used labels in LFA. Colloidal gold is inert and gives very perfect spherical particles. These particles have very high affinity toward biomolecules and can be easily functionalized. Optical properties of gold nanoparticles are dependent on size and shape. Size of particles can be tuned by use of suitable chemical additives. Their unique features include environment friendly preparation, high affinity toward proteins and biomolecules, enhanced stability, exceptionally higher values for charge transfer and good optical signaling. Optical signal of gold nanoparticles in colorimetric LFA can be amplified by deposition of silver, gold nanoparticles and enzymes.

In one embodiment, the label comprises colloidal nanoparticulate gold. In one embodiment, the gold particles have a diameter size in the range of about 20-55 nm, preferably 35-45 nm. According to the claimed invention, the LFI device comprises a first, labeled monoclonal antibody against TRAIL and a second mobile, labeled monoclonal antibody against CRP.

In another embodiment, the label does not comprise a gold nanoparticle.

***Magnetic particles and aggregates*:** Colored magnetic particles produce color at the test line which is measured by an optical strip reader but magnetic signals coming from magnetic particles can also be used as detection signals and recorded by a magnetic assay reader. Magnetic signals are stable for longer time compared to optical signals and they enhance sensitivity of LFA by 10 to 1000 folds.

***Fluorescent and luminescent materials*:** Fluorescent molecules are widely used in LFA as labels and the amount of fluorescence is used to quantitate the concentration of analyte in the sample. Detection of proteins is accomplished by using organic fluorophores such as rhodamine as labels in LFA.

Current developments in nanomaterial have headed to manufacture of quantum dots which display very unique electrical and optical properties. These semiconducting particles are not only water soluble but can also be easily combined with biomolecules because of closeness in dimensions. Owing to their unique optical properties, quantum dots have come up as a substitute to organic fluorescent dyes. Like gold nanoparticles QDs show size dependent optical properties and a broad spectrum of wavelengths can be monitored. Single light source is sufficient to excite quantum dots of all different sizes. QDs have high photo stability and absorption coefficients.

Upconverting phosphors (UCP) are characterized by their excitation in infra-red region and emission in high energy visible region. Compared to other fluorescent materials, they have a unique advantage of not showing any auto fluorescence. Because of their excitation in IR regions, they do not photo degrade biomolecules. A major advantage lies in their production from easily available bulk materials. Although difference in batch to batch preparation of UCP reporters can affect sensitivity of analysis in LFA, it was observed that they can enhance sensitivity of analytical signal by 10 to 100 folds compared to gold nanoparticles or colored latex beads, when analysis is carried out under same set of biological conditions.

***Enzymes:*** Enzymes are also employed as labels in LFA. But they increase one step in LFA which is application of suitable substrate after complete assay. This substrate will produce color at test and control lines as a result of enzymatic reaction. In case of enzymes, selection of suitable enzyme substrate combination is one necessary requirement in order to get a colored product for strip reader or electroactive product for electrochemical detection. In other words, sensitivity of detection is dependent on enzyme substrate combination.

***Colloidal carbon:*** Colloidal carbon is comparatively inexpensive label and its production can be easily scaled up. Because of their black color, carbon NPs can be easily detected with high sensitivity. Colloidal carbon can be functionalized with a large variety of biomolecules for detection of low and high molecular weight analytes.

### Additional polypeptides that may be measured with TRAIL:

Examples of polypeptides that may be measured together with TRAIL using the LFIA contemplated by the present inventors are those set forth in Table 2 herein below. The claimed invention provides a method of measuring the amount of TRAIL and CRP.

**Table 2**

| **Protein symbol** | **Full Gene Name** | **RefSeq DNA sequence** | **RefSeq proteins** |
|---|---|---|---|
| CRP | C-reactive protein, pentraxin-related | NC_000001.11 | NP_000558.2 |
| | | NT_004487.20 | |
| | | NC_018912.2 | |
| IP-10 | Chemokine (C-X-C motif) ligand 10 | NC_000004.12 | NP_001556.2 |
| | | NC_018915.2 | |
| | | NT_016354.20 | |
| IL1R/IL1R1/ IL1RA | Interleukin 1 receptor, type I | NC_000002.12 | NP_000868.1 |
| | | NT_005403.18 | NP_001275635.1 |
| | | NC_018913.2 | |
| Procalcitonin (PCT) | Calcitonin-related polypeptide alpha | NC_000011.10 | NP_001029124.1 |
| | | NC_018922.2 | NP_001029125.1 |
| | | NT_009237.19 | NP_001732.1 |
| SAA/SAA1 | Serum amyloid A1 | NC_000011.10 | NP_000322.2 |
| | | NC_018922.2 | NP_001 171477.1 |
| | | NT_009237.19 | NP_954630.1 |
| TREM1 | Triggering receptor expressed on myeloid cells 1 | NC_000006.12 | NP_001229518.1 |
| | | NT_007592.16 | NP_001229519.1 |
| | | NC_018917.2 | NP_061113.1 |
| TREM2 | Triggering receptor expressed on myeloid cells 2 | NC_000006.12 | NP_001258750.1 |
| | | NT_007592.16 | NP_061838.1 |
| | | NC_018917.2 | |
| RSAD2 | Radical S-adenosyl | NC_000002.12 | NP_542388.2 |
| | methionine domain containing 2 | NT_005334.17 | |
| | | NC_018913.2 | |
| NGAL | Lipocalin 2 | NC_000009.12 | NP_005555.2 |
| | | NC_018920.2 | |
| | | NT_008470.20 | |
| MMP8 | Matrix metallopeptidase 8 | NC_000011.10 | NP_001291370.1 |
| | | NT_033899.9 | NP_001291371.1 |
| | | NC_018922.2 | NP_002415.1 |
| MX1 | MX Dynamin-Like GTPase 1 | NC_000021.9 | NP_001138397.1 |
| | | NT_011512.12 | NP_001171517.1 |
| | | NC_018932.2 | NP_001269849.1 |
| | | | NP_002453.2 |

Other examples of polypeptides that may be measured using the LFIA of this aspect of the present invention include but are not limited to: OTOF, PI3, CYBRD1, EIF2AK2, CMPK2, IL1RA, IP10, Mac-2BP, B2M, BCA-1, CHI3L1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, CRP, SAA, TREM-1, PCT, IL-8, IL6, ARG1, ARPC2, ATP6V0B, BCA-1, BRI3BP, CCL19-MIP3b, CES1, CORO1A, HERC5, IFI6, IFIT3, KIAA0082, LIPT1, LRDD, MCP-2, PARP9, PTEN, QARS, RAB13, RPL34, SART3, TRIM22, UBE2N, XAF1 and ZBP1.

**IL1RA:** The protein encoded by this gene is a cytokine receptor that belongs to the interleukin 1 receptor family. Additional names of the gene include without limitations: CD121A, IL-1RT1, p80, CD121a antigen, CD121A, IL1R and IL1RA.

A representative RefSeq amino acid sequence of this protein is NP_000558.2. Representative RefSeq DNA sequences include: NC_000001.11, NT_004487.20, NC_018912.2.

**PCT:** Procalcitonin (PCT) is a peptide precursor of the hormone calcitonin.

A representative RefSeq amino acid sequence of this protein is NP_000558.2. Representative RefSeq DNA sequences include: NC_000001.11, NT_004487.20, NC_018912.2.

**SAA:** encodes a member of the serum amyloid A family of apolipoproteins.

A representative RefSeq amino acid sequence of this protein is NP_000558.2. Representative RefSeq DNA sequences include: NC_000001.11, NT_004487.20, NC_018912.2.

**CHP:** A representative RefSeq amino acid sequence of this protein is NP_009167.1. Representative RefSeq DNA sequences include: NC_000015.10, NT_010194.18, NC_018926.2.

**CMPK2:** A representative RefSeq amino acid sequence of this protein is NP_001243406.1, NP_001243407.1, NP_997198.2. Representative RefSeq DNA sequences include: NC_000002.12, NT_005334.17, NC_018913.2.

**CORO1C:** A representative RefSeq amino acid sequence of this protein is NP_001098707.1, NP_001263400.1, NP_055140.1. Representative RefSeq DNA sequences include: NC_000012.12, NT_029419.13, NC_018923.2.

**EIF2AK2:** Additional aliases include without limitation: PKR, PRKR, EIF2AK1, protein kinase, interferon-inducible double stranded RNA dependent, p68 kinase.

A representative RefSeq amino acid sequence of this protein is NP_001129123.1, NP_001129124.1, NP_002750.1. Representative RefSeq DNA sequences include:NC_000002.12, NT_022184.16, NC_018913.2.

**ISG15:** ISG15 ubiquitin-like modifier; additional aliases of ISG15 include without limitation G1P2, IFI15, IP17, UCRP and hUCRP.

A representative RefSeq amino acid sequence of this protein is NP_005092.1. Representative RefSeq DNA sequences include: NC_000001.11, NC_018912.2, NT_032977.10.

**RTN3:** A representative RefSeq amino acid sequence of this protein is NP_001252518.1, NP_001252519.1, NP_001252520.1, NP_006045.1, NP_958831.1, NP_958832.1, NP_958833.1. Representative RefSeq DNA sequences include: NC_000011.10, NT_167190.2, NC_018922.2.

**CD112:** This gene encodes a single-pass type I membrane glycoprotein with two Ig-like C2-type domains and an Ig-like V-type domain.

A representative RefSeq amino acid sequence of this protein is NP_001036189.1, NP_002847.1. Representative RefSeq DNA sequences include: NC_000019.10, NT_011109.17, NC_018930.2.

**CD134:** The protein encoded by this gene is a member of the TNF-receptor superfamily.

A representative RefSeq amino acid sequence of this protein is NP_003318.1. Representative RefSeq DNA sequences include: NC_000001.11, NT_004487.20, NC_018912.2.

**CD182:** The protein encoded by this gene is a member of the G-protein-coupled receptor family.

A representative RefSeq amino acid sequence of this protein is NP_001136269.1 or NP_001495.1. Representative RefSeq DNA sequences include: NC_000023.11, NT_011651.18, NC_018934.2.

**CD231:** The protein encoded by this gene is a member of the transmembrane 4 superfamily, also known as the tetraspanin family.

A representative RefSeq amino acid sequence of this protein is Representative RefSeq DNA sequences include: NC_000023.11, NC_018934.2, NT_079573.5.

**CD235a:** CD235a is the major intrinsic membrane protein of the erythrocyte.

A representative RefSeq amino acid sequence of this protein is NP_002090.4. Representative RefSeq DNA sequences include: NC_000004.12, NT_016354.20, NC_018915.2.

**CD335:** Representative RefSeq amino acid sequence of this protein are NP_001138929.2, NP_001138930.2, NP_001229285.1, NP_001229286.1 or NP_004820.2. Representative RefSeq DNA sequences include: NC_000019.10, NT_011109.17, NT_187693.1, NC_018930.2, NT_187671.1, NT_187674.1, NT_187675.1, NT_187676.1, NT_187677.1, NT_187683.

**CD337:** The protein encoded by this gene is a natural cytotoxicity receptor (NCR).

Representative RefSeq amino acid sequences of this protein are NP_001138938.1, NP_001138939.1, NP_667341.1. Representative RefSeq DNA sequences include: NC_000006.12, NT_007592.16, NT_167244.2, NT_113891.3, NT_167245.2, NT_167246.2, NT_167247.2, NT_167248.2, NT_167249.2, NC_018917.2.

**CD45:** The protein encoded by this gene is a member of the protein tyrosine phosphatase (PTP) family.

Representative RefSeq amino acid sequences of this protein are NP_001254727.1, NP_002829.3, NP_563578.2. Representative RefSeq DNA sequences include: NC_000001.11, NT_004487.20, NC_018912.2.

**CD49d:** The product of this gene belongs to the integrin alpha chain family of proteins.

A representative RefSeq amino acid sequences of this protein is NP_000876.3. Representative RefSeq DNA sequences include: NC_000002.12, NC_018913.2, NT_005403.18.

**CD66a:** This gene encodes a member of the carcinoembryonic antigen (CEA) gene family, which belongs to the immunoglobulin superfamily.

Representative RefSeq amino acid sequences of this protein are NP_001020083.1, NP_001171742.1, NP_001171744.1, NP_001171745.1, NP_001192273.1, NP_001703.2. Representative RefSeq DNA sequences include: NC_000019.10, NT_011109.17, NC_018930.2.

**CD66c:** Carcinoembryonic antigen (CEA; MIM 114890).

A representative RefSeq amino acid sequences of this protein is NP_002474.4. Representative RefSeq DNA sequences include: NC_000019.10, NT_011109.17, NC_018930.2.

**CD66d:** This gene encodes a member of the family of carcinoembryonic antigen-related cell adhesion molecules (CEACAMs).

Representative RefSeq amino acid sequences of this protein are NP_001264092.1, NP_001806.2. Representative RefSeq DNA sequences include: NC_000019.10, NC_018930.2, NT_011109.17.

**CD66e:** CD66e is a member of the CEACAM subfamily.

Representative RefSeq amino acid sequences of this protein are NP_001278413.1, NP_004354.3. Representative RefSeq DNA sequences include: NC_000019.10, NT_011109.17, NC_018930.2.

**CD84:** Representative RefSeq amino acid sequences of this protein are NP_001171808.1, NP_001171810.1, NP_001171811.1, NP_003865.1. Representative RefSeq DNA sequences include: NC_000001.11, NT_004487.20, NC_018912.2.

**EGFR:** The protein encoded by this gene is a transmembrane glycoprotein that is a member of the protein kinase superfamily.

Representative RefSeq amino acid sequences of this protein are NP_005219.2, NP_958439.1, NP_958440.1, NP_958441.1. Representative RefSeq DNA sequences include: NC_000007.14, NC_018918.2, NT_007819.18.

**GPR162:** Representative RefSeq amino acid sequences of this protein are NP_055264.1 or NP_062832. Representative RefSeq DNA sequences include: NC_000012.12, NC_018923.2, NT_009759.17.

**HLA-A:** HLA-A belongs to the HLA class I heavy chain paralogues.

Representative RefSeq amino acid sequences of this protein are NP_001229687.1 or NP_002107.3. Representative RefSeq DNA sequences include: NT_167247.2, NC_018917.2, NT_113891.3, NT_167244.2, NC_000006.12, NT_007592.16, NT_167245.2, NT_167246.2, NT_167248.2, NT_167249.2.

**HLA-B:** HLA-B belongs to the HLA class I heavy chain paralogues.

A representative RefSeq amino acid sequence of this protein is NP_005505.2. Representative RefSeq DNA sequences include: NT_167246.2, NT_167249.2, NT_167247.2, NC_000006.12, NT_007592.16, NT_113891.3, NT_167248.2, NC_018917.2.

**HLA-C:** HLA-C belongs to the HLA class I heavy chain paralogues.

Representative RefSeq amino acid sequences of this protein are NP_001229971.1, NP_002108.4. Representative RefSeq DNA sequences include: NT_113891.3, NC_000006.12, NC_018917.2, NT_007592.16, NT_167245.2, NT_167246.2, NT_167247.2, NT_167248.2, NT_167249.2.

**ITGAM:** This gene encodes the integrin alpha M chain.

Representative RefSeq amino acid sequences of this protein are NP_000623.2, NP_001139280.1. Representative RefSeq DNA sequences include: NC_000016.10, NT_187260.1, NC_018927.2.

**NRG1:** Representative RefSeq amino acid sequences of this protein are of NP_001153467.1, NP_001153468.1, NP_001153471.1, NP_001153473.1, NP_001153474.1, NP_001153476.1, NP_001153477.1, NP_001153479.1, NP_001153480.1, NP_004486.2, NP_039250.2, NP_039251.2, NP_039252.2, NP_039253.1, NP_039254.1, NP_039256.2, NP_039258.1. Representative RefSeq DNA sequences include:NC_000008.11, NT_167187.2, NC_018919.2.

**RAP1B:** GTP-binding protein that possesses intrinsic GTPase activity. Representative RefSeq amino acid sequences of this protein are NP_001010942.1, NP_001238846.1, NP_001238847.1, NP_001238850.1, NP_001238851.1, NP_056461.1. Representative RefSeq DNA sequences include: NC_000012.12, NC_018923.2, NT_029419.13.

**SELI:** This gene encodes a selenoprotein, which contains a selenocysteine (Sec) residue at its active site. A representative RefSeq amino acid sequence of this protein is NP_277040.1. Representative RefSeq DNA sequences include: NC_000002.12, NC_018913.2, NT_022184.16.

**SPINT2:** This gene encodes a transmembrane protein with two extracellular Kunitz domains Representative RefSeq amino acid sequences of this protein are NP_001159575.1 or NP_066925.1. Representative RefSeq DNA sequences include:NC_000019.10, NC_018930.2, NT_011109.17.

**EIF4B:** Representative RefSeq amino acid sequences of this protein are NP_001287750.1 or NP_001408.2. Representative RefSeq DNA sequences include: NC_000012.12, NT_029419.13, NC_018923.2.

**IFIT1:** Interferon-induced protein with tetratricopeptide repeats. Representative RefSeq amino acid sequences of this protein are NP_001257856.1, NP_001257857.1, NP_001257858.1, NP_001257859.1, NP_001539.3. Representative RefSeq DNA sequences include: NC_000010.11, NC_018921.2, NT_030059.14.

**IFITM3/IFITM2:** IFN-induced antiviral protein. A representative RefSeq amino acid sequence of this protein is NP_066362.2. Representative RefSeq DNA sequences include: NC_000011.10, NC_018922.2, NT_009237.19.

**RSAD2:** Radical S-adenosyl methionine domain containing 2; additional aliases of RSAD2 include without limitation 2510004L01Rik, cig33, cig5 and vig1. Representative RefSeq amino acid sequences of this protein are NP_001277482.1, NP_001277486.1, NP_001277558.1, NP_057083.2. Representative RefSeq DNA sequences include: NC_000002.12, NT_005334.17, NC_018913.2.

**ADIPOR1:** ADIPOR1 is a receptor for globular and full-length adiponectin (APM1). Representative RefSeq amino acid sequences of this protein are NP _001277 4821, NP_001277486.1, NP_001277558.1, NP_057083.2. Representative RefSeq DNA sequences include: NC_000001.11, NC_018912.2, NT_004487.20.

**CD15 (FUT4):** A representative RefSeq amino acid sequence of this protein is NP_002024.1. Representative RefSeq DNA sequences include: NC_000011.10, NC_018922.2, NT_033899.9.

**CD73:** Representative RefSeq amino acid sequences of this protein are NP_001191742.1 or NP_002517.1. Representative RefSeq DNA sequences include: NC_000006.12, NC_018917.2, NT_025741.16.

**CD8A:** The CD8 antigen is a cell surface glycoprotein. Representative RefSeq amino acid sequences of this protein are NP_001139345.1, NP_001759.3 or NP_741969.1. Representative RefSeq DNA sequences include: NC_000002.12, NC_018913.2, NT_022184.16.

**IFITM1:** Encodes an IFN-inducedantiviral protein. A representative RefSeq amino acid sequence of this protein is NP_003632.3. Representative RefSeq DNA sequences include: NC_000011.10, NC_018922.2, NT_009237.19.

**IFITM3:** Encodes an IFN-induced antiviral protein. A representative RefSeq amino acid sequence of this protein is NP_066362.2. Representative RefSeq DNA sequences include: NC_000011.10, NC_018922.2, NT_009237.19.

**IL7R:** The protein encoded by this gene is a receptor for interleukine 7 (IL7). A representative RefSeq amino acid sequence of this protein is NP_002176.2. Representative RefSeq DNA sequences include: NC_000005.10, NT_006576.17, NC_018916.2.

**LOC26010 (SPATS2L DNAPTP6):** Representative RefSeq amino acid sequences of this protein are NP_001093892.1, NP_001093893.1, NP_001093894.1, NP_001269664.1, NP_001269672.1, NP_001269673.1, NP_056350.2. RefSeq DNA sequence: NC_000002.12, NT_005403.18, NC_018913.2.

**TREM1:** Triggering receptor expressed on myeloid cells 1; additional aliases of TREM1 are CD354 and TREM-1. Representative RefSeq amino acid sequences of this protein are NP_001229518.1, NP_001229519.1, NP_061113.1. Representative RefSeq DNA sequences include: NC_000001.11, NT_004487.20, NC_018912.2.

**IL6:** A representative RefSeq amino acid sequences of this protein is NP_000591.1. Representative RefSeq DNA sequences include: NC_000007.14, NT_007819.18, NC_018918.2.

**IL7:** This gene encodes a cytokine. Representative RefSeq amino acid sequences of this protein are NP_000871.1, NP_001186815.1, NP_001186816.1, NP_001186817.1. Representative RefSeq DNA sequences include: NC_000008.11, NT_008183.20, NC_018919.2.

**ARG1:** Arginase. Representative RefSeq amino acid sequences of this protein are NP_000036.2 or NP_001231367.1. Representative RefSeq DNA sequences include: NC_000006.12, NT_025741.16, NC_018917.2.

**ARPC2:** This gene encodes one of seven subunits of the human Arp2/3 protein complex. Representative RefSeq amino acid sequences of this protein are NP_005722.1 or NP_690601.1. Representative RefSeq DNA sequences include: NC_000002.12, NT_005403.18, NC_018913.2.

**ATP6V0B:** H+-ATPase (vacuolar ATPase, V-ATPase) is an enzyme transporter. Representative RefSeq amino acid sequences of this protein are NP_001034546.1, NP_001281262.1 or NP_004038.1. Representative RefSeq DNA sequences include: NC_000001.11, NC_018912.2, NT_032977.10.

**BRI3BP:** A representative RefSeq amino acid sequence of this protein is NP_542193.3. Representative RefSeq DNA sequences include: NC_000012.12, NT_029419.13, NC_18923.2.

**CCL19:** A representative RefSeq amino acid sequence of this protein is NP_006265.1. Representative RefSeq DNA sequences include: NC_000009.12, NC_018920.2, NT_008413.19.

**CES1:** Representative RefSeq amino acid sequences of this protein are NP_001020365.1, NP_001020366.1 or NP_001257.4. Representative RefSeq DNA sequences include: NC_000016.10, NT_010498.16, NC_018927.2.

**CORO1A:** Representative RefSeq amino acid sequences of this protein are NP_001180262.1 or NP_009005.1. Representative RefSeq DNA sequences include: NC_000016.10, NT_187260.1, NC_018927.2.

**HERC5:** A representative RefSeq amino acid sequence of this protein is NP_057407.2. Representative RefSeq DNA sequences include: NC_000004.12, NT_016354.20, NC_018915.2.

**IFI6:** Representative RefSeq amino acid sequences of this protein are NP_002029.3, NP_075010.1, NP_075011.1. Representative RefSeq DNA sequences include: NC_000001.11, NC_018912.2, NT_032977.10.

**IFIT3:** Additional aliases of the protein include without limitation: interferon-induced protein with tetratricopeptide repeats 3, IFI60, ISG60 and Interferon-induced 60 kDa protein.

Representative RefSeq amino acid sequences of this protein are NP_001026853.1, NP_001276687.1, NP_001276688.1, NP_001540.2. Representative RefSeq DNA sequences include: NC_000010.11, NC_018921.2, NT_030059.14.

**MBOAT2:** Acyltransferase. A representative RefSeq amino acid sequence of this protein is NP_620154.2. Representative RefSeq DNA sequences include: NC_000002.12, NT_005334.17, NC_018913.2.

**MX1/MXA:** myxovirus (influenza virus) resistance 1; additional aliases of MX1 include without limitation IFI-78K, IFI78, MX and MxA. Representative RefSeq amino acid sequences of this protein are NP_001138397.1, NP_001171517.1, NP_001269849.1, NP_002453.2. Representative RefSeq DNA sequences include: NC_000021.9, NT_011512.12, NC_018932.2.

**OAS2:** This gene encodes a member of the 2-5A synthetase family. Representative RefSeq amino acid sequences of this protein are NP_001027903.1, NP_002526.2, NP_058197.2. Representative RefSeq DNA sequences include:NC_000012.12, NT_029419.13, NC_018923.2.

**KIAA0082 (FTSJD2):** S-adenosyl-L-methionine-dependent methyltransferase. A representative RefSeq amino acid sequence of this protein is NP_055865.1. Representative RefSeq DNA sequences include: NC_000006.12, NT_007592.16, NC_018917.2.

**LIPT1:** Representative RefSeq amino acid sequences of this protein are NP_001191759.1, NP_057013.1, NP_660198.1, NP_660199.1, NP_660200.1. Representative RefSeq DNA sequences include: NC_000002.12, NC_018913.2, NT_005403.18.

**LRDD:** Representative RefSeq amino acid sequences of this protein are NP_665893.2 or NP_665894.2. Representative RefSeq DNA sequences include: NC_000011.10, NT_009237.19, NC_018922.2.

**MCP-2:** This gene encodes a cytokine. A representative RefSeq amino acid sequence of this protein is NP_005614.2. Representative RefSeq DNA sequences include: NC_000017.11, NC_018928.2, NT_010783.16.

**PARP9:** Poly (ADP-ribose) polymerase (PARP). Representative RefSeq amino acid sequences of this protein are NP_001139574.1, NP_001139575.1, NP_001139576.1, NP_001139577.1, NP_001139578.1, NP_113646.2. Representative RefSeq DNA sequences include: NC_000003.12, NT_005612.17, NC_018914.2.

**PTEN:** Representative RefSeq amino acid sequences of this protein are NP_000305.3, NP_001291646.2, NP_001291647.1. Representative RefSeq DNA sequences include: NC_000010.11, NT_030059.14, N_018921.2.

**QARS:** Aminoacyl-tRNA synthetases catalyze the aminoacylation of tRNA by their cognate amino acid. Representative RefSeq amino acid sequences of this protein are NP_001259002.1 or NP_005042.1. Representative RefSeq DNA sequences include: NC_000003.12, NT_022517.19, NC_018914.2.

**RAB13:** Representative RefSeq amino acid sequences of this protein are NP_001258967.1, NP_002861.1. Representative RefSeq DNA sequences include: NC_000001.11, NC_018912.2, NT_004487.20.

**RPL22L1:** A representative RefSeq amino acid sequence of this protein is NP_001093115.1. Representative RefSeq DNA sequences include: NC_000003.12, NT_005612.17, NC_018914.2.

**RPL34:** The protein belongs to the L34E family of ribosomal proteins. Representative RefSeq amino acid sequences of this protein are NP_000986.2 or NP_296374.1. Representative RefSeq DNA sequences include: NC_00000-4.12, NT_016354.20, NC_018915.2.

**SART3:** The protein encoded by this gene is an RNA-binding nuclear protein. A representative RefSeq amino acid sequence of this protein is NP_055521.1. Representative RefSeq DNA sequences include: NC_000012.12, NT_029419.13, NC_018923.2.

**SSEA-1:** A representative RefSeq amino acid sequence of this protein is NP_002024.1. Representative RefSeq DNA sequences include: NC_000011.10, NC_018922.2, NT_033899.9.

**TRIM22:** Interferon-induced antiviral protein. Representative RefSeq amino acid sequences of this protein are NP_001186502.1 or NP_006065.2. Representative RefSeq DNA sequences include: NC_000011.10, NC_018922.2, NT_009237.19.

**UBE2N:** A representative RefSeq amino acid sequence of this protein is NP_003339.1. Representative RefSeq DNA sequences include: NC_000012.12, NT_029419.13, NC_018923.2.

**XAF1:** Representative RefSeq amino acid sequences of this protein are NP_059993.2 or NP_954590.1. Representative RefSeq DNA sequences include: NC_000017.11, NT_010718.17, NC_018928.2.

**ZBP1:** Representative RefSeq amino acid sequences of this protein are NP_001082.2 or NP_001259001.1. Representative RefSeq DNA sequences include: NC_000007.14, NT_007933.16, NC_018918.2.

**IL11:** The protein encoded by this gene is a member of the gp130 family of cytokines. Representative RefSeq amino acid sequences of this protein are NP_000632.1 or NP_001254647.1. Representative RefSeq DNA sequences include: NC_000019.10, NC_018930.2, NT_011109.17.

**I-TAC:** Additional names of the gene include without limitations: SCYB11, SCYB9B and CXCL11. Representative RefSeq amino acid sequences of this protein are NP_001289052.1, NP_005400.1. Representative RefSeq DNA sequences include: NC_000004.12, NC_018915.2, NT_016354.20.

**TNFR1:** Receptor for TNFSF2/TNF-alpha and homotrimeric TNFSF1/lymphotoxin-alpha. Additional names of the gene include without limitations: TNFRSF1A, TNFAR, p55, p60, CD120a antigen and CD120a antigen.

A representative RefSeq amino acid sequence of this protein is NP_003780.1. Representative RefSeq DNA sequences include: NC_000016.10, NT_010498.16, NC_018927.2.

**IL-8:** The protein encoded by this gene is a member of the CXC chemokine family. Additional aliases of IL-8 include without limitation: Interleukin 8, K60, CXCL8, SCYB8, GCP-1, TSG-1, MDNCF, b-ENAP, MONAP, alveolar macrophage chemotactic factor I, NAP-1, beta endothelial cell-derived neutrophil activating peptide, GCP1, beta-thromboglobulin-like protein, LECT, chemokine (C-X-C motif) ligand 8, LUCT, emoctakin, LYNAP, interleukin-8, NAF, lung giant cell carcinoma-derived chemotactic protein, NAP1, lymphocyte derived neutrophil activating peptide, IL-8, neutrophil-activating peptide 1, Granulocyte chemotactic protein 1, small inducible cytokine subfamily B, member 8, Monocyte-derived neutrophil chemotactic factor, tumor necrosis factor-induced gene 1, Monocyte-derived neutrophil-activating peptide, Emoctakin, T-cell chemotactic factor,C-X-C motif chemokine 8, 3-10C,Neutrophil-activating protein 1, AMCF-I and Protein 3-10C.

A representative RefSeq amino acid sequence of this protein is NP_000575.1. Representative RefSeq DNA sequences include: NC_000004.12, NC_018915.2, NT_016354.20.

**HP** - This gene encodes a preproprotein, which is processed to yield both alpha and beta chains, which subsequently combine as a tetramer to produce haptoglobin. Representative RefSeq amino acid sequences of this protein are NP_001119574.1, or NP_005134.1. Representative RefSeq DNA sequences include: NC_000016.10, NT_010498.16, NC_018927.2.

As used herein the term "about" refers to ± 10 %.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

Throughout this application, various embodiments of this invention may be presented in a range format. It should be understood that the description in range format is merely for convenience and brevity.

Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

In the context of the present invention, the following abbreviations may be used: ANC = Absolute neutrophil count; ANN = Artificial neural networks; AUC = Area under the receiver operating curve; BP = Bordetella pertussis; CHF = Congestive heart failure; CI = Confidence interval; CID = Congenital immune deficiency; CLL = Chronic lymphocytic leukemia; CMV = Cytomegalovirus; CNS = Central nervous system; COPD = Chronic obstructive pulmonary disease; CP = Chlamydophila pneumonia; CRP = C-reactive protein; CSF = Cerebrospinal fluid; CV = Coefficient of variation; DOR = Diagnostic odds ratio; EBV = Epstein bar virus; eCRF = Electronic case reportform; ED = Emergency department, ELISA = Enzyme-linked immunosorbent assay; FDR = False discovery rate; FMF = Familial Mediterranean fever; G-CSF = Granulocyte colony-stimulating factor; GM-CSF = Granulocyte-macrophage colony-stimulating factor; HBV = Hepatitis B virus; HCV = Hepatitis C virus; HI = Haemophilus influenza; HIV = Human immunodeficiency virus; IDE = Infectious disease experts; IL = Interleukin; IRB = institutional review board; IVIG = Intravenous immunoglobulin; KNN = K-nearest neighbors; LP = Legionella pneumophila; LR+ = Positive likelihood ratio; LR- = Negative likelihood ratio; LRTI = Lower respiratory tract infections; mAb = Monoclonal antibodies; MDD = Minimum detectable dose; MDS = Myelodysplastic syndrome; MP = Mycoplasma pneumonia; MPD = Myeloproliferative disease; NPV = Negative predictive value; PCT = Procalcitonin; PED = Pediatric emergency department; PPV = Positive predictive value; QA = Quality assurance; RSV = Respiratory syncytial virus; RV = Rhinovirus; SIRS = systemic inflammatory syndrome; SP = Streptococcus pneumonia; STARD = Standards for Reporting of Diagnostic Accuracy; SVM = Support vector machine; TNF = Tumor necrosis factor; URTI = Upper respiratory tract infection; UTI = Urinary tract infection; WBC = White blood cell; WS = Wilcoxon rank-sum.

In the context of the present invention, the following statistical terms may be used:
"TP" is true positive, means positive test result that accurately reflects the tested-for activity. For example in the context of the present invention a TP, is for example but not limited to, truly classifying a bacterial infection as such.

"TN" is true negative, means negative test result that accurately reflects the tested-for activity. For example in the context of the present invention a TN, is for example but not limited to, truly classifying a viral infection as such.

"FN" is false negative, means a result that appears negative but fails to reveal a situation. For example in the context of the present invention a FN, is for example but not limited to, falsely classifying a bacterial infection as a viral infection.

"FP" is false positive, means test result that is erroneously classified in a positive category. For example in the context of the present invention a FP, is for example but not limited to, falsely classifying a viral infection as a bacterial infection.

"Sensitivity" is calculated by TP/(TP+FN) or the true positive fraction of disease subjects.

"Specificity" is calculated by TN/(TN+FP) or the true negative fraction of non-disease or normal subjects.

"Total accuracy" is calculated by (TN + TP)/(TN + FP +TP + FN).

"Positive predictive value" or "PPV" is calculated by TP/(TP+FP) or the true positive fraction of all positive test results. It is inherently impacted by the prevalence of the disease and pre-test probability of the population intended to be tested.

"Negative predictive value" or "NPV" is calculated by TN/(TN + FN) or the true negative fraction of all negative test results. It also is inherently impacted by the prevalence of the disease and pre-test probability of the population intended to be tested. See, e.g., O'Marcaigh AS, Jacobson RM, "Estimating The Predictive Value Of A Diagnostic Test, How To Prevent Misleading Or Confusing Results," Clin. Ped. 1993, 32(8): 485-491, which discusses specificity, sensitivity, and positive and negative predictive values of a test, e.g., a clinical diagnostic test.
"MCC" (Mathews Correlation coefficient) is calculated as follows: MCC = (TP * TN - FP * FN) / {(TP + FN) * (TP + FP) * (TN + FP) * (TN + FN)}^0.5
where TP, FP, TN, FN are true-positives, false-positives, true-negatives, and false-negatives, respectively. Note that MCC values range between -1 to +1, indicating completely wrong and perfect classification, respectively. An MCC of 0 indicates random classification. MCC has been shown to be a useful for combining sensitivity and specificity into a single metric (Baldi, Brunak et al. 2000). It is also useful for measuring and optimizing classification accuracy in cases of unbalanced class sizes (Baldi, Brunak et al. 2000).

"Accuracy" refers to the degree of conformity of a measured or calculated quantity (a test reported value) to its actual (or true) value. Clinical accuracy relates to the proportion of true outcomes (true positives (TP) or true negatives (TN) versus misclassified outcomes (false positives (FP) or false negatives (FN)), and may be stated as a sensitivity, specificity, positive predictive values (PPV) or negative predictive values (NPV), Mathews correlation coefficient (MCC), or as a likelihood, odds ratio, Receiver Operating Characteristic (ROC) curve, Area Under the Curve (AUC) among other measures.

"Analytical accuracy" refers to the reproducibility and predictability of the measurement process itself, and may be summarized in such measurements as coefficients of variation (CV), Pearson correlation, and tests of concordance and calibration of the same samples or controls with different times, users, equipment and/or reagents. These and other considerations in evaluating new biomarkers are also summarized in Vasan, 2006.

"Performance" is a term that relates to the overall usefulness and quality of a diagnostic or prognostic test, including, among others, clinical and analytical accuracy, other analytical and process characteristics, such as use characteristics (e.g., stability, ease of use), health economic value, and relative costs of components of the test. Any of these factors may be the source of superior performance and thus usefulness of the test, and may be measured by appropriate "performance metrics," such as AUC and MCC, time to result, shelf life, etc. as relevant.

By "statistically significant", it is meant that the alteration is greater than what might be expected to happen by chance alone (which could be a "false positive"). Statistical significance can be determined by any method known in the art. Commonly used measures of significance include the p-value, which presents the probability of obtaining a result at least as extreme as a given data point, assuming the data point was the result of chance alone. A result is often considered highly significant at a p-value of 0.05 or less.

The term "determinant" as used herein refers to a disease associated parameter or biomarker.

## Claims

1. A method of measuring the amount of TNF-related apoptosis-inducing ligand (TRAIL) polypeptide and CRP polypeptide in a fluid sample of a subject in need thereof, the method comprising:
(a) flowing said sample through a lateral flow immunoassay (LFI) device which comprises a first mobile, labeled monoclonal antibody against said TRAIL; a second mobile labeled monoclonal antibody against said CRP; a first immobilized, non-labeled monoclonal antibody against said TRAIL; and a second immobilized, non-labeled monoclonal antibody against said CRP; and
(b) determining the amount of said TRAIL polypeptide and said CRP polypeptide at a test band of said device.

2. The method of claim 1, wherein said labeled antibody comprises a label selected from the group consisting of a chromogen, a catalyst, a gold nanoparticle, a fluorescent agent, a chemiluminescent agent, a dye particle, and a latex particle tagged with a detector reagent.

3. The method of any one of claims 1-2, wherein said lateral flow immunoassay device is capable of detecting at least one additional polypeptide, said polypeptide being selected from the group consisting of IP10, NGAL, MMP8, IL1RA, OTOF, PI3, CYBRD1, EIF2AK2, CMPK2, Mac-2BP, B2M, BCA-1, CHI3L1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, SAA, TREM-1, PCT, IL-8, IL6, ARG1, ARPC2, ATP6V0B, BCA-1, BRI3BP, CCL19-MIP3b, CES1, CORO1A, HERC5, IFI6, IFIT3, KIAA0082, LIPT1, LRDD, MCP-2, PARP9, PTEN, QARS, RAB13, RPL34, SART3, TRIM22, UBE2N, XAF1 and ZBP1.

4. The method of claim 3, wherein said at least one additional polypeptide is IP10.

5. The method of claim 1, wherein said first labeled antibody and said second labeled antibody are comprised on a single lateral flow test strip.

6. The method of claim 1, wherein said first labeled antibody is comprised on a first lateral flow test strip and said second labeled antibody is comprised on a second lateral flow test strip.

7. The method of claim 4, wherein said first or said second lateral flow test strip comprises a labeled antibody against IP10.

8. The method of claims 5 or 6, wherein said lateral flow immunoassay device comprises an additional lateral flow test strip which comprises a labeled antibody against said IP10.

9. The method of claim 4, wherein said lateral flow immunoassay device comprises a first lateral flow test strip which comprises a labeled antibody against said TRAIL, a second lateral flow test strip which comprises a labeled antibody against said CRP and a third lateral flow test strip which comprises a labeled antibody against said IP10.

10. The method of any one of claims 1-9, wherein said fluid sample comprises a blood sample or a fraction thereof.

11. A method of distinguishing between a bacterial and viral infection in a subject comprising:
(a) measuring the amount of TRAIL polypeptide and CRP polypeptide in a fluid sample of the subject according to the method of any one of claims 1-10; and
(b) diagnosing the subj ect with a bacterial or viral infection according to the amount of said TRAIL polypeptide and said CRP polypeptide.

12. The method of claim 11, wherein said fluid sample has been taken from the subject no more than two days following symptoms of an infection.

13. A lateral flow test strip comprising a conjugate pad which comprises:
(i) a first mobile, labeled monoclonal antibody against TRAIL polypeptide and a detection membrane comprising an immobilized, non-labeled monoclonal antibody against said TRAIL polypeptide; and
(ii) a second mobile, labeled monoclonal antibody against CRP polypeptide and a detection membrane comprising an immobilized, non-labeled monoclonal antibody against said CRP polypeptide.

14. A kit comprising the lateral flow test strip of claim 13, a sample collector and a diluent.

## Patentansprüche

1. Ein Verfahren zur Messung der Menge von TNF-Related-Apoptosis-Inducing-Ligand(TRAIL)-Polypeptid und CRP-Polypeptid in einer Flüssigkeitsprobe eines Individuums, bei dem dies erforderlich ist, wobei das Verfahren Folgendes beinhaltet:
(a) Fließenlassen der genannten Probe durch eine Lateral-Flow-Immunoassay(LFI)-Vorrichtung, die Folgendes beinhaltet: einen ersten mobilen, markierten monoklonalen Antikörper gegen das genannte TRAIL; einen zweiten mobilen markierten monoklonalen Antikörper gegen das genannte CRP; einen ersten immobilisierten, nicht markierten monoklonalen Antikörper gegen das genannte TRAIL; und einen zweiten immobilisierten, nicht markierten monoklonalen Antikörper gegen das genannte CRP; und
(b) Bestimmen der Menge des genannten TRAIL-Polypeptids und genannten CRP-Polypeptids an einer Testbande der genannten Vorrichtung.

2. Verfahren nach Anspruch 1, wobei der genannte markierte Antikörper eine Markierung beinhaltet, die aus der Gruppe ausgewählt ist, die aus einem chromogenen Mittel, einem Katalysator, einem Goldnanopartikel, einem fluoreszierenden Mittel, einem chemilumineszierenden Mittel, einem Farbmittelteilchen und einem Latexteilchen, gekennzeichnet mit einem Detektorreagenz, besteht.

3. Verfahren nach einem der Ansprüche 1-2, wobei die genannte Lateral-Flow-Immunoassay-Vorrichtung in der Lage ist, mindestens ein zusätzliches Polypeptid zu detektieren, wobei das genannte Polypeptid aus der Gruppe ausgewählt ist, die aus IP10, NGAL, MMP8, IL1RA, OTOF, PI3, CYBRD1, EIF2AK2, CMPK2, Mac-2BP, B2M, BCA-1, CHI3L1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, SAA, TREM-1, PCT, IL-8, IL6, ARG1, ARPC2, ATP6V0B, BCA-1, BRI3BP, CCL19-MIP3b, CES1, CORO1A, HERC5, IFI6, IFIT3, KIAA0082, LIPT1, LRDD, MCP-2, PARP9, PTEN, QARS, RAB13, RPL34, SART3, TRIM22, UBE2N, XAF1 und ZBP1 besteht.

4. Verfahren nach Anspruch 3, wobei das genannte mindestens eine zusätzliche Polypeptid IP10 ist.

5. Verfahren nach Anspruch 1, wobei der genannte erste markierte Antikörper und genannte zweite markierte Antikörper auf einem einzigen Lateral-Flow-Teststreifen enthalten sind.

6. Verfahren nach Anspruch 1, wobei der genannte erste markierte Antikörper auf einem ersten Lateral-Flow-Teststreifen enthalten ist und der genannte zweite markierte Antikörper auf einem zweiten Lateral-Flow-Teststreifen enthalten ist.

7. Verfahren nach Anspruch 4, wobei der genannte erste oder genannte zweite Lateral-Flow-Teststreifen einen markierten Antikörper gegen IP10 beinhaltet.

8. Verfahren nach Ansprüchen 5 oder 6, wobei die genannte Lateral-Flow-Immunoassay-Vorrichtung einen zusätzlichen Lateral-Flow-Teststreifen beinhaltet, der einen markierten Antikörper gegen das genannte IP10 beinhaltet.

9. Verfahren nach Anspruch 4, wobei die genannte Lateral-Flow-Immunoassay-Vorrichtung Folgendes beinhaltet: einen ersten Lateral-Flow-Teststreifen, der einen markierten Antikörper gegen das genannte TRAIL beinhaltet, einen zweiten Lateral-Flow-Teststreifen, der einen markierten Antikörper gegen das genannte CRP beinhaltet, und einen dritten Lateral-Flow-Teststreifen, der einen markierten Antikörper gegen das genannte IP10 beinhaltet.

10. Verfahren nach einem der Ansprüche 1-9, wobei die genannte Flüssigkeitsprobe eine Blutprobe oder eine Fraktion davon beinhaltet.

11. Ein Verfahren zur Unterscheidung zwischen einer bakteriellen und viralen Infektion bei einem Individuum, das Folgendes beinhaltet:
(a) Messen der Menge von TRAIL-Polypeptid und CRP-Polypeptid in einer Flüssigkeitsprobe des Individuums entsprechend dem Verfahren nach einem der Ansprüche 1-10; und
(b) Diagnostizieren einer bakteriellen oder viralen Infektion bei dem Individuum entsprechend der Menge des genannten TRAIL-Polypeptids und des genannten CRP-Polypeptids.

12. Verfahren nach Anspruch 11, wobei die genannte Flüssigkeitsprobe dem Individuum nicht mehr als zwei Tage nach Infektionssymptomen entnommen wurde.

13. Ein Lateral-Flow-Teststreifen, der ein Konjugat-Pad beinhaltet, das Folgendes beinhaltet:
(i) einen ersten mobilen, markierten monoklonalen Antikörper gegen TRAIL-Polypeptid und eine Detektionsmembran, die einen immobilisierten, nicht markierten monoklonalen Antikörper gegen das genannte TRAIL-Polypeptid beinhaltet; und
(ii) einen zweiten mobilen, markierten monoklonalen Antikörper gegen CRP-Polypeptid und eine Detektionsmembran, die einen immobilisierten, nicht markierten monoklonalen Antikörper gegen das genannte CRP-Polypeptid beinhaltet.

14. Ein Kit, das den Lateral-Flow-Teststreifen nach Anspruch 13, einen Probensammler und ein Verdünnungsmittel beinhaltet.

## Revendications

1. Procédé de mesure de la quantité de polypeptide TRAIL (ligand induisant l'apoptose de la famille ou TNF) et de polypeptide CRP (protéine C-réactive) dans un échantillon de fluide d'un sujet qui en a besoin, le procédé comprenant :
(a) un écoulement dudit échantillon à travers un dispositif de test immunochromatographique qui comprend un premier anticorps monoclonal mobile, marqué, dirigé contre ledit TRAIL ; un deuxième anticorps monoclonal mobile, marqué, dirigé contre ladite CRP ; un premier anticorps monoclonal immobilisé, non marqué, dirigé contre ledit TRAIL ; et un deuxième anticorps monoclonal immobilisé, non marqué, dirigé contre ladite CRP ; et
(b) une détermination de la quantité dudit polypeptide TRAIL et dudit polypeptide CRP dans une bandelette de test dudit dispositif.

2. Procédé selon la revendication 1, dans lequel ledit anticorps marqué comprend un marqueur sélectionné dans le groupe constitué d'un chromogène, d'un catalyseur, d'une nanoparticule d'or, d'un agent fluorescent, d'un agent chimioluminescent, d'une particule de colorant, et d'une particule de latex marquée avec un réactif de détection.

3. Procédé selon l'une quelconque des revendications 1 ou 2, dans lequel ledit dispositif de test immunochromatographique est capable de détecter au moins un polypeptide supplémentaire, ledit polypeptide étant sélectionné dans le groupe constitué de IP10, NGAL, MMP8, IL1RA, OTOF, PI3, CYBRD1, EIF2AK2, CMPK2, Mac-2BP, B2M, BCA-1, CHI3L1, Eotaxin, IL1a, MCP, CD62L, VEGFR2, CHP, CMPK2, CORO1C, EIF2AK2, ISG15, RPL22L1, RTN3, CD112, CD134, CD182, CD231, CD235A, CD335, CD337, CD45, CD49D, CD66A/C/D/E, CD73, CD84, EGFR, GPR162, HLA-A/B/C, ITGAM, NRG1, RAP1B, SELI, SPINT2, SSEA1, IL1, I-TAC, TNFR1, IFITM3, IFIT3, EIF4B, IFIT1, LOC26010, MBOAT2, MX1, OAS2, RSAD2, ADIPOR1, CD15, CD8A, IFITM1, IL7, SAA, TREM-1, PCT, IL-8, IL6, ARG1, ARPC2, ATP6V0B, BCA-1, BRI3BP, CCL19-MIP3b, CES1, CORO1A, HERC5, IF16, IFIT3, KIAA0082, LIPT1, LRDD, MCP-2, PARP9, PTEN, QARS, RAB13, RPL34, SART3, TRIM22, UBE2N, XAF1 et ZBP1.

4. Procédé selon la revendication 3, dans lequel ledit au moins un polypeptide supplémentaire est IP10.

5. Procédé selon la revendication 1, dans lequel ledit premier anticorps marqué et ledit deuxième anticorps marqué sont présents sur une seule bandelette de test immunochromatographique.

6. Procédé selon la revendication 1, dans lequel ledit premier anticorps marqué est présent sur une première bandelette de test immunochromatographique et ledit deuxième anticorps marqué est présent sur une deuxième bandelette de test immunochromatographique.

7. Procédé selon la revendication 4, dans lequel ladite première ou ladite deuxième bandelette de test immunochromatographique comprend un anticorps marqué dirigé contre IP10.

8. Procédé selon les revendications 5 ou 6, dans lequel ledit dispositif de test immunochromatographique comprend une bandelette de test immunochromatographique supplémentaire qui comprend un anticorps marqué dirigé contre ledit IP10.

9. Procédé selon la revendication 4, dans lequel ledit dispositif de test immunochromatographique comprend une première bandelette de test immunochromatographique qui comprend un anticorps marqué dirigé contre ledit TRAIL, une deuxième bandelette de test immunochromatographique qui comprend un anticorps marqué dirigé contre ladite CRP, et une troisième bandelette de test immunochromatographique qui comprend un anticorps marqué dirigé contre ledit IP10.

10. Procédé selon l'une quelconque des revendications 1 à 9, dans lequel ledit échantillon de fluide comprend un échantillon de sang ou une fraction de celui-ci.

11. Procédé permettant de faire la distinction entre une infection bactérienne et une infection virale chez un sujet, comprenant :
(a) une mesure de la quantité de polypeptide TRAIL et de polypeptide CRP dans un échantillon de fluide du sujet en suivant le procédé selon l'une quelconque des revendications 1 à 10 ; et
(b) un diagnostic chez le sujet d'une infection bactérienne ou d'une infection virale en fonction de la quantité dudit polypeptide TRAIL et dudit polypeptide CRP.

12. Procédé selon la revendication 11, dans lequel ledit échantillon de fluide a été prélevé sur le sujet pas plus de deux jours suivant les symptômes d'une infection.

13. Bandelette de test immunochromatographique comprenant un tampon conjugué qui comprend :
(i) un premier anticorps monoclonal mobile, marqué, dirigé contre le polypeptide TRAIL, et une membrane de détection comprenant un anticorps monoclonal immobilisé, non marqué, dirigé contre ledit polypeptide TRAIL ; et
(ii) un deuxième anticorps monoclonal mobile, marqué, dirigé contre le polypeptide CRP, et une membrane de détection comprenant un anticorps monoclonal immobilisé, non marqué, dirigé contre ledit polypeptide CRP.

14. Trousse comprenant la bandelette de test immunochromatographique selon la revendication 13, un collecteur d'échantillons et un diluant.
